# EUROPEAN PATENT APPLICATION

(11) **EP 3 257 944 A1**
(43) Date of publication of application: **20.12.2017**
(21) Application number: 16177993.9
(22) Date of filing: 05.07.2016
(51) Int. Cl.: C12N 15/82, A01H 5/00

(54) **TOMATO PLANTS HAVING ALTERATIONS IN THE DMR6 GENE**

(30) Priority: 14.06.2016 US 201662349831 P
(71) Applicant: Nunhems B.V., 6083 AB Nunhem (NL)
(72) Inventor: The designation of the inventor has not yet been filed
(74) Representative: BIP Patents

(57) **Abstract**

The present invention relates to a cultivated plant of the species *Solanum lycopersicum* comprising a *dmr6* allele encoding a mutant DMR6 protein.

SEQUENCE LISTING

<110>  Nunhems B.V.

<120>  Tomato plants having non-transgenic alterations in the DMR6 gene

<130>  BCS 16-8012 EP

<160>  11

<170>  PatentIn version 3.5

<210>  1
<211>  337
<212>  PRT
<213>  Lycopersicon esculentum

<400>  1

Met Glu Thr Lys Val Ile Ser Ser Gly Ile Asn His Ser Thr Leu Pro
1               5                   10                  15

Gln Ser Tyr Ile Arg Pro Glu Ser Asp Arg Pro Arg Leu Ser Glu Val
            20                  25                  30

Val Asp Cys Glu Asn Val Pro Ile Ile Asp Leu Ser Cys Gly Asp Gln
        35                  40                  45

Ala Gln Ile Ile Arg Gln Ile Gly Glu Ala Cys Gln Thr Tyr Gly Phe
    50                  55                  60

Phe Gln Val Ile Asn His Gly Val Pro Lys Glu Val Val Glu Lys Met
65                  70                  75                  80

Leu Gly Val Ala Gly Glu Phe Phe Asn Leu Pro Val Glu Glu Lys Leu
                85                  90                  95

Lys Leu Tyr Ser Asp Asp Pro Ser Lys Thr Met Arg Leu Ser Thr Ser
            100                 105                 110

Phe Asn Val Lys Lys Glu Thr Val His Asn Trp Arg Asp Tyr Leu Arg
        115                 120                 125

Leu His Cys Tyr Pro Leu Glu Lys Tyr Ala Pro Glu Trp Pro Ser Asn
    130                 135                 140

Pro Ser Ser Phe Arg Glu Ile Val Ser Arg Tyr Cys Arg Glu Ile Arg
145                 150                 155                 160

Gln Leu Gly Phe Arg Leu Glu Glu Ala Ile Ala Glu Ser Leu Gly Leu
                165                 170                 175

Asp Lys Glu Cys Ile Lys Asp Val Leu Gly Glu Gln Gly Gln His Met
            180                 185                 190

Ala Ile Asn Tyr Tyr Pro Pro Cys Pro Gln Pro Glu Leu Thr Tyr Gly
        195                 200                 205

Leu Pro Ala His Thr Asp Pro Asn Ser Leu Thr Ile Leu Leu Gln Asp
    210                 215                 220

Leu Gln Val Ala Gly Leu Gln Val Leu Lys Asp Gly Lys Trp Leu Ala
225                 230                 235                 240

Val Lys Pro Gln Pro Asp Ala Phe Val Ile Asn Leu Gly Asp Gln Leu
                245                 250                 255

Gln Ala Val Ser Asn Gly Lys Tyr Arg Ser Val Trp His Arg Ala Ile
            260                 265                 270

Val Asn Ser Asp Gln Ala Arg Met Ser Val Ala Ser Phe Leu Cys Pro
        275                 280                 285

Cys Asp Ser Ala Lys Ile Ser Ala Pro Lys Leu Leu Thr Glu Asp Gly
    290                 295                 300

Ser Pro Val Ile Tyr Gln Asp Phe Thr Tyr Ala Glu Tyr Tyr Asn Lys
305                 310                 315                 320

Phe Trp Ser Arg Asn Leu Asp Gln Gln His Cys Leu Glu Leu Phe Lys
                325                 330                 335

Asn

<210>  2
<211>  337
<212>  PRT
<213>  Lycopersicon esculentum

<400>  2

Met Glu Thr Lys Val Ile Ser Ser Gly Ile Asn His Ser Thr Leu Pro
1               5                   10                  15

Gln Ser Tyr Ile Arg Pro Glu Ser Asp Arg Pro Arg Leu Ser Glu Val
            20                  25                  30

Val Asp Cys Glu Asn Val Pro Ile Ile Asp Leu Ser Cys Gly Asp Gln
        35                  40                  45

Ala Gln Ile Ile Arg Gln Ile Gly Glu Ala Cys Gln Thr Tyr Gly Phe
    50                  55                  60

Phe Gln Val Ile Asn His Gly Val Pro Lys Glu Val Val Glu Lys Met
65                  70                  75                  80

Leu Gly Val Ala Gly Glu Phe Phe Asn Leu Pro Val Glu Glu Lys Leu
                85                  90                  95

Lys Leu Tyr Ser Asp Asp Pro Ser Lys Thr Met Arg Leu Ser Thr Ser
            100                 105                 110

Phe Asn Val Lys Lys Glu Thr Val His Asn Trp Arg Asp Tyr Leu Arg
        115                 120                 125

Leu His Cys Tyr Pro Leu Glu Lys Tyr Ala Pro Glu Trp Pro Ser Asn
    130                 135                 140

Pro Ser Ser Phe Arg Glu Ile Val Ser Arg Tyr Cys Arg Glu Ile Arg
145                 150                 155                 160

Gln Leu Gly Phe Lys Leu Glu Glu Ala Ile Ala Glu Ser Leu Gly Leu
                165                 170                 175

Asp Lys Glu Cys Ile Lys Asp Val Leu Gly Glu Gln Gly Gln His Met
            180                 185                 190

Ala Ile Asn Tyr Tyr Pro Pro Cys Pro Gln Pro Glu Leu Thr Tyr Gly
        195                 200                 205

Leu Pro Ala His Thr Asp Pro Asn Ser Leu Thr Ile Leu Leu Gln Asp
    210                 215                 220

Leu Gln Val Ala Gly Leu Gln Val Leu Lys Asp Gly Lys Trp Leu Ala
225                 230                 235                 240

Val Lys Pro Gln Pro Asp Ala Phe Val Ile Asn Leu Gly Asp Gln Leu
                245                 250                 255

Gln Ala Val Ser Asn Gly Lys Tyr Arg Ser Val Trp His Arg Ala Ile
            260                 265                 270

Val Asn Ser Asp Gln Ala Arg Met Ser Val Ala Ser Phe Leu Cys Pro
        275                 280                 285

Cys Asp Ser Ala Lys Ile Ser Ala Pro Lys Leu Leu Thr Glu Asp Gly
    290                 295                 300

Ser Pro Val Ile Tyr Gln Asp Phe Thr Tyr Ala Glu Tyr Tyr Asn Lys
305                 310                 315                 320

Phe Trp Ser Arg Asn Leu Asp Gln Gln His Cys Leu Glu Leu Phe Lys
                325                 330                 335

Asn

<210>  3
<211>  337
<212>  PRT
<213>  Lycopersicon esculentum

<400>  3

Met Glu Thr Lys Val Ile Ser Ser Gly Ile Asn His Ser Thr Leu Pro
1               5                   10                  15

Gln Ser Tyr Ile Arg Pro Glu Ser Asp Arg Pro Arg Leu Ser Glu Val
            20                  25                  30

Val Asp Cys Glu Asn Val Pro Ile Ile Asp Leu Ser Cys Gly Asp Gln
        35                  40                  45

Ala Gln Ile Ile Arg Gln Ile Gly Glu Ala Cys Gln Thr Tyr Gly Phe
    50                  55                  60

Phe Gln Val Ile Asn His Gly Val Pro Lys Glu Val Val Glu Lys Met
65                  70                  75                  80

Leu Gly Val Ala Gly Glu Phe Phe Asn Leu Pro Val Glu Glu Lys Leu
                85                  90                  95

Lys Leu Tyr Ser Asp Asp Pro Ser Lys Thr Met Arg Leu Ser Thr Ser
            100                 105                 110

Phe Asn Val Lys Lys Glu Thr Val His Asn Trp Arg Asp Tyr Leu Arg
        115                 120                 125

Leu His Cys Tyr Pro Leu Glu Lys Tyr Ala Pro Glu Trp Pro Ser Asn
    130                 135                 140

Pro Ser Ser Phe Arg Glu Ile Val Ser Arg Tyr Cys Arg Glu Ile Arg
145                 150                 155                 160

Gln Leu Gly Phe Arg Leu Glu Glu Ala Ile Ala Glu Ser Leu Gly Leu
                165                 170                 175

Asp Lys Glu Cys Ile Lys Asp Val Phe Gly Glu Gln Gly Gln His Met
            180                 185                 190

Ala Ile Asn Tyr Tyr Pro Pro Cys Pro Gln Pro Glu Leu Thr Tyr Gly
        195                 200                 205

Leu Pro Ala His Thr Asp Pro Asn Ser Leu Thr Ile Leu Leu Gln Asp
    210                 215                 220

Leu Gln Val Ala Gly Leu Gln Val Leu Lys Asp Gly Lys Trp Leu Ala
225                 230                 235                 240

Val Lys Pro Gln Pro Asp Ala Phe Val Ile Asn Leu Gly Asp Gln Leu
                245                 250                 255

Gln Ala Val Ser Asn Gly Lys Tyr Arg Ser Val Trp His Arg Ala Ile
            260                 265                 270

Val Asn Ser Asp Gln Ala Arg Met Ser Val Ala Ser Phe Leu Cys Pro
        275                 280                 285

Cys Asp Ser Ala Lys Ile Ser Ala Pro Lys Leu Leu Thr Glu Asp Gly
    290                 295                 300

Ser Pro Val Ile Tyr Gln Asp Phe Thr Tyr Ala Glu Tyr Tyr Asn Lys
305                 310                 315                 320

Phe Trp Ser Arg Asn Leu Asp Gln Gln His Cys Leu Glu Leu Phe Lys
                325                 330                 335

Asn

<210>  4
<211>  1014
<212>  DNA
<213>  Lycopersicon esculentum

<400>  4
atggaaacca aagttatttc tagcggaatc aaccactcta ctcttcctca aagttacatc      60

cgacccgaat ccgatagacc acgtctatcg gaagtggtcg attgtgaaaa tgttccaata     120

attgacttaa gttgcggaga tcaagctcaa ataattcgtc aaattggaga agcttgtcaa     180

acttatggtt tctttcaggt aattaatcat ggtgtaccaa aggaagttgt agagaaaatg     240

ctaggggtag ctggggaatt tttcaattta ccagtagaag agaaactaaa attatattca     300

gatgatcctt caaagaccat gagattatca acaagtttta atgttaaaaa ggagacagtt     360

cataattgga gagattatct cagacttcat tgttatcctc tagagaagta tgctcctgaa 420

tggccttcta atccatcatc tttcagggaa atcgtgagca gatattgcag ggaaattcgt 480

caactcggat ttagattaga agaagccata gcagaaagcc tggggttaga taaagagtgt 540

ataaaagatg tattgggtga acaaggacaa catatggcta tcaattatta tcctccttgt 600

ccacaaccag aacttactta tgggcttccg gcccatactg atccaaattc acttacaatt 660

cttcttcaag acttgcaagt tgcgggtctt caagttctta aagatggcaa atggttagct 720

gtaaaacctc aacctgacgc ctttgtcatt aatcttgggg atcaattgca ggcagtaagt 780

aacggtaagt acagaagtgt atggcatcga gctattgtga attcagatca agctaggatg 840

tcagtggctt cgtttctatg tccgtgtgat agcgcgaaaa tcagtgcacc aaagctgctg 900

acagaagatg gatctccagt gatttatcaa gactttacgt atgctgagta ttacaacaag 960

ttctggagca ggaatttgga ccagcaacat tgtttggaac ttttcaagaa ctaa 1014

<210> 5
<211> 4472
<212> DNA
<213> Lycopersicon esculentum

<220>
<221> exon
<222> (1)..(198)
<223> Exon 1

<220>
<221> exon
<222> (282)..(529)
<223> Exon 2

<220>
<221> exon
<222> (3776)..(4100)
<223> Exon 3

<220>
<221> exon
<222> (4230)..(4472)
<223> Exon 4

<400> 5
atg gaa acc aaa gtt att tct agc gga atc aac cac tct act ctt cct 48
Met Glu Thr Lys Val Ile Ser Ser Gly Ile Asn His Ser Thr Leu Pro
1               5                   10                  15

caa agt tac atc cga ccc gaa tcc gat aga cca cgt cta tcg gaa gtg 96
Gln Ser Tyr Ile Arg Pro Glu Ser Asp Arg Pro Arg Leu Ser Glu Val
            20                  25                  30

gtc gat tgt gaa aat gtt cca ata att gac tta agt tgc gga gat caa 144
Val Asp Cys Glu Asn Val Pro Ile Ile Asp Leu Ser Cys Gly Asp Gln
        35                  40                  45

gct caa ata att cgt caa att gga gaa gct tgt caa act tat ggt ttc 192

Ala Gln Ile Ile Arg Gln Ile Gly Glu Ala Cys Gln Thr Tyr Gly Phe
    50                  55                  60

ttt cag gttcttatcg ttggattcat gaattaataa atatttttcg attattaacg      248
Phe Gln
65

ataaaaataa taatcgactt tactactttt tag gta att aat cat ggt gta cca      302
                                     Val Ile Asn His Gly Val Pro
                                                 70

aag gaa gtt gta gag aaa atg cta ggg gta gct ggg gaa ttt ttc aat      350
Lys Glu Val Val Glu Lys Met Leu Gly Val Ala Gly Glu Phe Phe Asn
    75                  80                  85

tta cca gta gaa gag aaa cta aaa tta tat tca gat gat cct tca aag      398
Leu Pro Val Glu Glu Lys Leu Lys Leu Tyr Ser Asp Asp Pro Ser Lys
90                  95                  100                 105

acc atg aga tta tca aca agt ttt aat gtt aaa aag gag aca gtt cat      446
Thr Met Arg Leu Ser Thr Ser Phe Asn Val Lys Lys Glu Thr Val His
                110                 115                 120

aat tgg aga gat tat ctc aga ctt cat tgt tat cct cta gag aag tat      494
Asn Trp Arg Asp Tyr Leu Arg Leu His Cys Tyr Pro Leu Glu Lys Tyr
            125                 130                 135

gct cct gaa tgg cct tct aat cca tca tct ttc ag  gtaaacacca      539
Ala Pro Glu Trp Pro Ser Asn Pro Ser Ser Phe Arg
        140                 145

ttaattaact gtttaactac tttctttatg ttaatataca ttgctatctg agtcatactt      599

caattattat tatcattata tacgtcaacc actttgtctg tatagtaatg catggattgg      659

aagtaacctc aaataatata tcgtatgaga tgttgtcaag tatctaagtt gtttacgcgt      719

ataagagaaa tacgaggctc aaactgagaa gtgttgcatc ctttaaaata agtgcatgta      779

catcacatat attaaaaaat agaacatgtg tcagatgcgt gtcagatgtt tgtcaatatt      839

atagaatgta aatatcctca ataaaatttc gttaaagaac tacctaatat atatatatat      899

atataaaaat aaatagttct taattaggta ataatttatt tctttctaag aagagtcaac      959

tcattcaaaa tatatattta taaaaacata aaaataaaaa aagaagaaga tgattctcta      1019

ataattagaa ctacctaatc tacctagcta acctttcaat tgatttttct agaatgcatg      1079

ttccctttta atgtcccaac catttttaat ttcttgacct tttcttttcc tccttctttc      1139

atatttcaac tttgtgttta agaagtattc cctcaatttt gtattaatta aacttgtaaa      1199

atattatata tttattaata aaaatatttt aatctagatt tttaaaaaaa ttcaatttat      1259

actataagaa aacttaaaat ttaattaata taaaatagag tttaatatta aatttgattt      1319

tggtcaccta tatttcccac aagtatgttc aaatattgga tgacgatttt tttcatagat      1379

gtggccttga cctagtcaat aattttgttg cgattcctca acttccgata acaaaaaata      1439

taatagccac ataaattaga gtaaccataa ggaagaaaaa tgtagaattt tgatttttgg      1499

aaaaaaaaat aaagtttaat gtcagagatt gacagaaata agcatgtgag gcgaggccat 1559

atttgactat ttcaagatgt catagtaatc ctatttttct ttttcaaaaa taaaatgttg 1619

tctcaagatg tagtaccata tagaatatat taattaactt attatatatt acgtcatgga 1679

tgacaaattt gatgacgcca tcatagtgcc tatatttgtc cattaatcga cacataagca 1739

atgagtgcgt catcctagtg atgacgtcaa taccgttacg taagccgaca aaagtgatag 1799

gtgatgggtg gccttaactc atgattcggg tcgactcaaa atcggatttc tttattagaa 1859

taatttagaa taatttttat ttattaatta catcaatatt taatatttat tttatatcac 1919

aaatttaaaa aattattata ccttactaat atattatgtt taagtaaaaa atgttgcata 1979

aatatgaaga aatagtatta attgcctttc tttttttggc aagctctact gtttcttttt 2039

ttccttccta tttttttttt tttggtttaa agcaagtact gtttctattt gctacacaaa 2099

aagtactttt aacctcttta aatattcact cgctcgcttt ttatcgatcc aattatatgt 2159

taattattat attttgatac gaatttttct tttattataa aaagagatac ataataacct 2219

taaatcaaat aatttgttaa taactttaaa cgataataaa taaatatatt aataaaaaat 2279

gcttaaatat ttatcgacat tagttaaata tcgttaagtt taatatcgta aagcctttaa 2339

atacatattt gtcctctgaa attttttgat atagcaaata cataatttcc tttaaaatta 2399

aattcatttt aatccatccc agcccataaa tgacttatac tgcttttgac aatttcagac 2459

ccatcatttt cgttcttgtc gtacaatttc cccagttctt ttcattccca agtgtcaata 2519

tccaaaattt tatgattgga ctaatatata gttatattgc ataaatttat caaatttcca 2579

tattataagg cttgaatcta gacttttgaa tgtgggcata gatattttat accttctatc 2639

tacctcttgc tcaaaaagta aataaaagag tgctcaataa agaaattata taagtttttt 2699

taattgaaat ttgtagatct tttctttaaa gtgaagaata gtcgagaaac gaattaattt 2759

tctagtgtaa catgttgcgc tataattaag ccgaacaagg acaaacaaca ttaaataaac 2819

aagataaata taatattatt agttttatgt acccccataa caaagtgtct cgacccttga 2879

atgtgtaccc aaaaagtaac agtgcccttt gacaatctcg aatgttgaaa aatacaatta 2939

acacagcaaa tgaccatatt acccttggtt acgaagcaaa catgtcgggg aaaacctgct 2999

tctagacttc tctatatagg ttattcgtct aacaattatt gtccaataaa caagtagttt 3059

atgctctctc cgtttcattt tagatatcat cttttgaatt tgaagagtca aattagttta 3119

cgtttgatcg taaaattttc atagatcttt taaacatgtt aaattatcaa ttattatgac 3179

tcatagtact ttttacgtag tttacaaata tataaatttc attccaaaaa attaaagatt 3239

ttatgcgcaa atttttaatt aaaattaaac tcaaaacgta tagatgtcat aaatattgga 3299

acaaattgtt tatttttact cattatagac tcgtagcgat attttacata agtatttaat 3359

ttttctgttt gtatactaat gagtaatgat gacaattaag ttatatttta aacaaaaaaa 3419

atgaaggtgg gtaagcaatg aggatctgta acgtgaaaga taagaatatg ctgattttgt 3479

tgagtttcat ggctaaaaaa acgcgttttt tcaggattaa tttagcaaat gaaagtgact 3539

tgcaacataa attcaaatgg cataacgtaa gccaagccaa tgaataatgg ggcttttagt 3599

tcaattatag agcatttgat aaaataataa aaagtgagtg gagatgttga attagaaagc 3659

cagtagtgcc ttttttggat cccaactgat tacgcccaaa acactctcac tagattaggg 3719

tacataataa aatgcattaa tattcgaaga attaatgtaa atagtcgttg ttacag g 3776

gaa atc gtg agc aga tat tgc agg gaa att cgt caa ctc gga ttt aga 3824
Glu Ile Val Ser Arg Tyr Cys Arg Glu Ile Arg Gln Leu Gly Phe Arg
150                 155                 160                 165

tta gaa gaa gcc ata gca gaa agc ctg ggg tta gat aaa gag tgt ata 3872
Leu Glu Glu Ala Ile Ala Glu Ser Leu Gly Leu Asp Lys Glu Cys Ile
                170                 175                 180

aaa gat gta ttg ggt gaa caa gga caa cat atg gct atc aat tat tat 3920
Lys Asp Val Leu Gly Glu Gln Gly Gln His Met Ala Ile Asn Tyr Tyr
            185                 190                 195

cct cct tgt cca caa cca gaa ctt act tat ggg ctt ccg gcc cat act 3968
Pro Pro Cys Pro Gln Pro Glu Leu Thr Tyr Gly Leu Pro Ala His Thr
        200                 205                 210

gat cca aat tca ctt aca att ctt ctt caa gac ttg caa gtt gcg ggt 4016
Asp Pro Asn Ser Leu Thr Ile Leu Leu Gln Asp Leu Gln Val Ala Gly
    215                 220                 225

ctt caa gtt ctt aaa gat ggc aaa tgg tta gct gta aaa cct caa cct 4064
Leu Gln Val Leu Lys Asp Gly Lys Trp Leu Ala Val Lys Pro Gln Pro
230                 235                 240                 245

gac gcc ttt gtc att aat ctt ggg gat caa ttg cag gtaaaccaca 4110
Asp Ala Phe Val Ile Asn Leu Gly Asp Gln Leu Gln
                250                 255

actccttcaa atcgttataa gttaataata ataatcttta tgcaactcat tggagatatt 4170

aggtagtcgt tggattattg tatgaaattc gaatctaata gaaaattgtg attgtgcag 4229

gca gta agt aac ggt aag tac aga agt gta tgg cat cga gct att gtg 4277
Ala Val Ser Asn Gly Lys Tyr Arg Ser Val Trp His Arg Ala Ile Val
        260                 265                 270

aat tca gat caa gct agg atg tca gtg gct tcg ttt cta tgt ccg tgt 4325
Asn Ser Asp Gln Ala Arg Met Ser Val Ala Ser Phe Leu Cys Pro Cys
    275                 280                 285

gat agc gcg aaa atc agt gca cca aag ctg ctg aca gaa gat gga tct 4373
Asp Ser Ala Lys Ile Ser Ala Pro Lys Leu Leu Thr Glu Asp Gly Ser
290                 295                 300                 305

cca gtg att tat caa gac ttt acg tat gct gag tat tac aac aag ttc 4421
Pro Val Ile Tyr Gln Asp Phe Thr Tyr Ala Glu Tyr Tyr Asn Lys Phe
                310                 315                 320

tgg agc agg aat ttg gac cag caa cat tgt ttg gaa ctt ttc aag aac 4469
Trp Ser Arg Asn Leu Asp Gln Gln His Cys Leu Glu Leu Phe Lys Asn

325                 330                 335

taa                                                                   4472

<210>  6
<211>  1014
<212>  DNA
<213>  Lycopersicon esculentum

<400>  6
atggaaacca aagttatttc tagcggaatc aaccactcta ctcttcctca aagttacatc      60

cgacccgaat ccgatagacc acgtctatcg gaagtggtcg attgtgaaaa tgttccaata     120

attgacttaa gttgcggaga tcaagctcaa ataattcgtc aaattggaga agcttgtcaa     180

acttatggtt tctttcaggt aattaatcat ggtgtaccaa aggaagttgt agagaaaatg     240

ctaggggtag ctggggaatt tttcaattta ccagtagaag agaaactaaa attatattca     300

gatgatcctt caaagaccat gagattatca acaagtttta atgttaaaaa ggagacagtt     360

cataattgga gagattatct cagacttcat tgttatcctc tagagaagta tgctcctgaa     420

tggccttcta atccatcatc tttcagggaa atcgtgagca gatattgcag ggaaattcgt     480

caactcggat ttaaattaga agaagccata gcagaaagcc tggggttaga taaagagtgt     540

ataaaagatg tattgggtga acaaggacaa catatggcta tcaattatta tcctccttgt     600

ccacaaccag aacttactta tgggcttccg gcccatactg atccaaattc acttacaatt     660

cttcttcaag acttgcaagt tgcgggtctt caagttctta aagatggcaa atggttagct     720

gtaaaacctc aacctgacgc ctttgtcatt aatcttgggg atcaattgca ggcagtaagt     780

aacggtaagt acagaagtgt atggcatcga gctattgtga attcagatca agctaggatg     840

tcagtggctt cgtttctatg tccgtgtgat agcgcgaaaa tcagtgcacc aaagctgctg     900

acagaagatg gatctccagt gatttatcaa gactttacgt atgctgagta ttacaacaag     960

ttctggagca ggaatttgga ccagcaacat tgtttggaac ttttcaagaa ctaa          1014

<210>  7
<211>  4472
<212>  DNA
<213>  Lycopersicon esculentum

<220>
<221>  exon
<222>  (1)..(198)
<223>  exon 1

<220>
<221>  exon
<222>  (282)..(529)
<223>  exon 2

<220>

<221>  exon
<222>  (3776)..(4100)
<223>  exon 3

<220>
<221>  exon
<222>  (4230)..(4472)
<223>  exon 4

<400>  7
atg gaa acc aaa gtt att tct agc gga atc aac cac tct act ctt cct      48
Met Glu Thr Lys Val Ile Ser Ser Gly Ile Asn His Ser Thr Leu Pro
1               5                   10                  15

caa agt tac atc cga ccc gaa tcc gat aga cca cgt cta tcg gaa gtg      96
Gln Ser Tyr Ile Arg Pro Glu Ser Asp Arg Pro Arg Leu Ser Glu Val
            20                  25                  30

gtc gat tgt gaa aat gtt cca ata att gac tta agt tgc gga gat caa      144
Val Asp Cys Glu Asn Val Pro Ile Ile Asp Leu Ser Cys Gly Asp Gln
        35                  40                  45

gct caa ata att cgt caa att gga gaa gct tgt caa act tat ggt ttc      192
Ala Gln Ile Ile Arg Gln Ile Gly Glu Ala Cys Gln Thr Tyr Gly Phe
    50                  55                  60

ttt cag gttcttatcg ttggattcat gaattaataa atatttttcg attattaacg      248
Phe Gln
65

ataaaaataa taatcgactt tactactttt tag gta att aat cat ggt gta cca      302
                                     Val Ile Asn His Gly Val Pro
                                                 70

aag gaa gtt gta gag aaa atg cta ggg gta gct ggg gaa ttt ttc aat      350
Lys Glu Val Val Glu Lys Met Leu Gly Val Ala Gly Glu Phe Phe Asn
    75                  80                  85

tta cca gta gaa gag aaa cta aaa tta tat tca gat gat cct tca aag      398
Leu Pro Val Glu Glu Lys Leu Lys Leu Tyr Ser Asp Asp Pro Ser Lys
90                  95                  100                 105

acc atg aga tta tca aca agt ttt aat gtt aaa aag gag aca gtt cat      446
Thr Met Arg Leu Ser Thr Ser Phe Asn Val Lys Lys Glu Thr Val His
                110                 115                 120

aat tgg aga gat tat ctc aga ctt cat tgt tat cct cta gag aag tat      494
Asn Trp Arg Asp Tyr Leu Arg Leu His Cys Tyr Pro Leu Glu Lys Tyr
            125                 130                 135

gct cct gaa tgg cct tct aat cca tca tct ttc ag  gtaaacacca      539
Ala Pro Glu Trp Pro Ser Asn Pro Ser Ser Phe Arg
        140                 145

ttaattaact gtttaactac tttctttatg ttaatataca ttgctatctg agtcatactt      599

caattattat tatcattata tacgtcaacc actttgtctg tatagtaatg catggattgg      659

aagtaacctc aaataatata tcgtatgaga tgttgtcaag tatctaagtt gtttacgcgt      719

ataagagaaa tacgaggctc aaactgagaa gtgttgcatc ctttaaaata agtgcatgta      779

catcacatat attaaaaaat agaacatgtg tcagatgcgt gtcagatgtt tgtcaatatt      839

atagaatgta aatatcctca ataaaatttc gttaaagaac tacctaatat atatatatat 899

atataaaaat aaatagttct taattaggta ataatttatt tctttctaag aagagtcaac 959

tcattcaaaa tatatattta taaaaacata aaaataaaaa aagaagaaga tgattctcta 1019

ataattagaa ctacctaatc tacctagcta acctttcaat tgatttttct agaatgcatg 1079

ttccctttta atgtcccaac catttttaat ttcttgacct tttcttttcc tccttctttc 1139

atatttcaac tttgtgttta agaagtattc cctcaatttt gtattaatta aacttgtaaa 1199

atattatata tttattaata aaaatatttt aatctagatt tttaaaaaaa ttcaatttat 1259

actataagaa aacttaaaat ttaattaata taaaatagag tttaatatta aatttgattt 1319

tggtcaccta tatttcccac aagtatgttc aaatattgga tgacgatttt tttcatagat 1379

gtggccttga cctagtcaat aattttgttg cgattcctca acttccgata acaaaaaata 1439

taatagccac ataaattaga gtaaccataa ggaagaaaaa tgtagaattt tgatttttgg 1499

aaaaaaaaat aaagtttaat gtcagagatt gacagaaata agcatgtgag gcgaggccat 1559

atttgactat ttcaagatgt catagtaatc ctatttttct ttttcaaaaa taaaatgttg 1619

tctcaagatg tagtaccata tagaatatat taattaactt attatatatt acgtcatgga 1679

tgacaaattt gatgacgcca tcatagtgcc tatatttgtc cattaatcga cacataagca 1739

atgagtgcgt catcctagtg atgacgtcaa taccgttacg taagccgaca aaagtgatag 1799

gtgatgggtg gccttaactc atgattcggg tcgactcaaa atcggatttc tttattagaa 1859

taatttagaa taatttttat ttattaatta catcaatatt taatatttat tttatatcac 1919

aaatttaaaa aattattata ccttactaat atattatgtt taagtaaaaa atgttgcata 1979

aatatgaaga aatagtatta attgcctttc tttttttggc aagctctact gtttcttttt 2039

ttccttccta tttttttttt tttggtttaa agcaagtact gtttctattt gctacacaaa 2099

aagtactttt aacctcttta aatattcact cgctcgcttt ttatcgatcc aattatatgt 2159

taattattat attttgatac gaatttttct tttattataa aaagagatac ataataacct 2219

taaatcaaat aatttgttaa taactttaaa cgataataaa taaatatatt aataaaaaat 2279

gcttaaatat ttatcgacat tagttaaata tcgttaagtt taatatcgta aagcctttaa 2339

atacatattt gtcctctgaa attttttgat atagcaaata cataatttcc tttaaaatta 2399

aattcatttt aatccatccc agcccataaa tgacttatac tgcttttgac aatttcagac 2459

ccatcatttt cgttcttgtc gtacaatttc cccagttctt ttcattccca agtgtcaata 2519

tccaaaattt tatgattgga ctaatatata gttatattgc ataaatttat caaatttcca 2579

tattataagg cttgaatcta gacttttgaa tgtgggcata gatattttat accttctatc 2639

tacctcttgc tcaaaaagta aataaaagag tgctcaataa agaaattata taagtttttt 2699

taattgaaat ttgtagatct tttctttaaa gtgaagaata gtcgagaaac gaattaattt 2759

tctagtgtaa catgttgcgc tataattaag ccgaacaagg acaaacaaca ttaaataaac 2819

aagataaata taatattatt agttttatgt acccccataa caaagtgtct cgacccttga 2879

atgtgtaccc aaaaagtaac agtgcccttt gacaatctcg aatgttgaaa aatacaatta 2939

acacagcaaa tgaccatatt acccttggtt acgaagcaaa catgtcgggg aaaacctgct 2999

tctagacttc tctatatagg ttattcgtct aacaattatt gtccaataaa caagtagttt 3059

atgctctctc cgtttcattt tagatatcat cttttgaatt tgaagagtca aattagttta 3119

cgtttgatcg taaaattttc atagatcttt taaacatgtt aaattatcaa ttattatgac 3179

tcatagtact ttttacgtag tttacaaata tataaatttc attccaaaaa attaaagatt 3239

ttatgcgcaa atttttaatt aaaattaaac tcaaaacgta tagatgtcat aaatattgga 3299

acaaattgtt tatttttact cattatagac tcgtagcgat attttacata agtatttaat 3359

ttttctgttt gtatactaat gagtaatgat gacaattaag ttatatttta aacaaaaaaa 3419

atgaaggtgg gtaagcaatg aggatctgta acgtgaaaga taagaatatg ctgattttgt 3479

tgagtttcat ggctaaaaaa acgcgttttt tcaggattaa tttagcaaat gaaagtgact 3539

tgcaacataa attcaaatgg cataacgtaa gccaagccaa tgaataatgg ggcttttagt 3599

tcaattatag agcatttgat aaaataataa aaagtgagtg gagatgttga attagaaagc 3659

cagtagtgcc ttttttggat cccaactgat tacgcccaaa acactctcac tagattaggg 3719

tacataataa aatgcattaa tattcgaaga attaatgtaa atagtcgttg ttacag g 3776

gaa atc gtg agc aga tat tgc agg gaa att cgt caa ctc gga ttt aaa 3824
Glu Ile Val Ser Arg Tyr Cys Arg Glu Ile Arg Gln Leu Gly Phe Lys
150                 155                 160                 165

tta gaa gaa gcc ata gca gaa agc ctg ggg tta gat aaa gag tgt ata 3872
Leu Glu Glu Ala Ile Ala Glu Ser Leu Gly Leu Asp Lys Glu Cys Ile
                170                 175                 180

aaa gat gta ttg ggt gaa caa gga caa cat atg gct atc aat tat tat 3920
Lys Asp Val Leu Gly Glu Gln Gly Gln His Met Ala Ile Asn Tyr Tyr
            185                 190                 195

cct cct tgt cca caa cca gaa ctt act tat ggg ctt ccg gcc cat act 3968
Pro Pro Cys Pro Gln Pro Glu Leu Thr Tyr Gly Leu Pro Ala His Thr
        200                 205                 210

gat cca aat tca ctt aca att ctt ctt caa gac ttg caa gtt gcg ggt 4016
Asp Pro Asn Ser Leu Thr Ile Leu Leu Gln Asp Leu Gln Val Ala Gly
    215                 220                 225

ctt caa gtt ctt aaa gat ggc aaa tgg tta gct gta aaa cct caa cct 4064
Leu Gln Val Leu Lys Asp Gly Lys Trp Leu Ala Val Lys Pro Gln Pro
230                 235                 240                 245

gac gcc ttt gtc att aat ctt ggg gat caa ttg cag gtaaaccaca 4110
Asp Ala Phe Val Ile Asn Leu Gly Asp Gln Leu Gln
                250                 255

actccttcaa atcgttataa gttaataata ataatcttta tgcaactcat tggagatatt 4170

aggtagtcgt tggattattg tatgaaattc gaatctaata gaaaattgtg attgtgcag 4229

gca gta agt aac ggt aag tac aga agt gta tgg cat cga gct att gtg 4277
Ala Val Ser Asn Gly Lys Tyr Arg Ser Val Trp His Arg Ala Ile Val
        260                 265                 270

aat tca gat caa gct agg atg tca gtg gct tcg ttt cta tgt ccg tgt 4325
Asn Ser Asp Gln Ala Arg Met Ser Val Ala Ser Phe Leu Cys Pro Cys
    275                 280                 285

gat agc gcg aaa atc agt gca cca aag ctg ctg aca gaa gat gga tct 4373
Asp Ser Ala Lys Ile Ser Ala Pro Lys Leu Leu Thr Glu Asp Gly Ser
290                 295                 300                 305

cca gtg att tat caa gac ttt acg tat gct gag tat tac aac aag ttc 4421
Pro Val Ile Tyr Gln Asp Phe Thr Tyr Ala Glu Tyr Tyr Asn Lys Phe
                310                 315                 320

tgg agc agg aat ttg gac cag caa cat tgt ttg gaa ctt ttc aag aac 4469
Trp Ser Arg Asn Leu Asp Gln Gln His Cys Leu Glu Leu Phe Lys Asn
            325                 330                 335

taa 4472

<210> 8
<211> 1014
<212> DNA
<213> Lycopersicon esculentum

<400> 8
atggaaacca aagttatttc tagcggaatc aaccactcta ctcttcctca aagttacatc 60

cgacccgaat ccgatagacc acgtctatcg gaagtggtcg attgtgaaaa tgttccaata 120

attgacttaa gttgcggaga tcaagctcaa ataattcgtc aaattggaga agcttgtcaa 180

acttatggtt tctttcaggt aattaatcat ggtgtaccaa aggaagttgt agagaaaatg 240

ctaggggtag ctggggaatt tttcaattta ccagtagaag agaaactaaa attatattca 300

gatgatcctt caaagaccat gagattatca acaagtttta atgttaaaaa ggagacagtt 360

cataattgga gagattatct cagacttcat tgttatcctc tagagaagta tgctcctgaa 420

tggccttcta atccatcatc tttcagggaa atcgtgagca gatattgcag ggaaattcgt 480

caactcggat ttagattaga agaagccata gcagaaagcc tggggttaga taaagagtgt 540

ataaaagatg tatttggtga acaaggacaa catatggcta tcaattatta tcctccttgt 600

ccacaaccag aacttactta tgggcttccg gcccatactg atccaaattc acttacaatt 660

cttcttcaag acttgcaagt tgcgggtctt caagttctta aagatggcaa atggttagct 720

gtaaaacctc aacctgacgc ctttgtcatt aatcttgggg atcaattgca ggcagtaagt 780

aacggtaagt acagaagtgt atggcatcga gctattgtga attcagatca agctaggatg 840

tcagtggctt cgtttctatg tccgtgtgat agcgcgaaaa tcagtgcacc aaagctgctg 900

acagaagatg gatctccagt gatttatcaa gactttacgt atgctgagta ttacaacaag 960

ttctggagca ggaatttgga ccagcaacat tgtttggaac ttttcaagaa ctaa 1014

<210> 9
<211> 4472
<212> DNA
<213> Lycopersicon esculentum

<220>
<221> exon
<222> (1)..(198)
<223> exon 1

<220>
<221> exon
<222> (282)..(529)
<223> exon 2

<220>
<221> exon
<222> (3776)..(4100)
<223> exon 3

<220>
<221> exon
<222> (4230)..(4472)
<223> exon 4

<400> 9
atg gaa acc aaa gtt att tct agc gga atc aac cac tct act ctt cct 48
Met Glu Thr Lys Val Ile Ser Ser Gly Ile Asn His Ser Thr Leu Pro
1               5                   10                  15

caa agt tac atc cga ccc gaa tcc gat aga cca cgt cta tcg gaa gtg 96
Gln Ser Tyr Ile Arg Pro Glu Ser Asp Arg Pro Arg Leu Ser Glu Val
            20                  25                  30

gtc gat tgt gaa aat gtt cca ata att gac tta agt tgc gga gat caa 144
Val Asp Cys Glu Asn Val Pro Ile Ile Asp Leu Ser Cys Gly Asp Gln
        35                  40                  45

gct caa ata att cgt caa att gga gaa gct tgt caa act tat ggt ttc 192
Ala Gln Ile Ile Arg Gln Ile Gly Glu Ala Cys Gln Thr Tyr Gly Phe
    50                  55                  60

ttt cag gttcttatcg ttggattcat gaattaataa atatttttcg attattaacg 248
Phe Gln
65

ataaaaataa taatcgactt tactactttt tag gta att aat cat ggt gta cca 302
Val Ile Asn His Gly Val Pro
            70

aag gaa gtt gta gag aaa atg cta ggg gta gct ggg gaa ttt ttc aat 350
Lys Glu Val Val Glu Lys Met Leu Gly Val Ala Gly Glu Phe Phe Asn
    75                  80                  85

tta cca gta gaa gag aaa cta aaa tta tat tca gat gat cct tca aag 398
Leu Pro Val Glu Glu Lys Leu Lys Leu Tyr Ser Asp Asp Pro Ser Lys

90                  95                  100                 105

acc atg aga tta tca aca agt ttt aat gtt aaa aag gag aca gtt cat      446
Thr Met Arg Leu Ser Thr Ser Phe Asn Val Lys Lys Glu Thr Val His
                110                 115                 120

aat tgg aga gat tat ctc aga ctt cat tgt tat cct cta gag aag tat      494
Asn Trp Arg Asp Tyr Leu Arg Leu His Cys Tyr Pro Leu Glu Lys Tyr
            125                 130                 135

gct cct gaa tgg cct tct aat cca tca tct ttc ag  gtaaacacca           539
Ala Pro Glu Trp Pro Ser Asn Pro Ser Ser Phe Arg
        140                 145

ttaattaact gtttaactac tttctttatg ttaatataca ttgctatctg agtcatactt    599

caattattat tatcattata tacgtcaacc actttgtctg tatagtaatg catggattgg    659

aagtaacctc aaataatata tcgtatgaga tgttgtcaag tatctaagtt gtttacgcgt    719

ataagagaaa tacgaggctc aaactgagaa gtgttgcatc ctttaaaata agtgcatgta    779

catcacatat attaaaaaat agaacatgtg tcagatgcgt gtcagatgtt tgtcaatatt    839

atagaatgta aatatcctca ataaaatttc gttaaagaac tacctaatat atatatatat    899

atataaaaat aaatagttct taattaggta ataatttatt tctttctaag aagagtcaac    959

tcattcaaaa tatatattta taaaaacata aaaataaaaa aagaagaaga tgattctcta   1019

ataattagaa ctacctaatc tacctagcta acctttcaat tgatttttct agaatgcatg   1079

ttccctttta atgtcccaac catttttaat ttcttgacct tttcttttcc tccttctttc   1139

atatttcaac tttgtgttta agaagtattc cctcaatttt gtattaatta aacttgtaaa   1199

atattatata tttattaata aaaatatttt aatctagatt tttaaaaaaa ttcaatttat   1259

actataagaa aacttaaaat ttaattaata taaaatagag tttaatatta aatttgattt   1319

tggtcaccta tatttcccac aagtatgttc aaatattgga tgacgatttt tttcatagat   1379

gtggccttga cctagtcaat aattttgttg cgattcctca acttccgata acaaaaaata   1439

taatagccac ataaattaga gtaaccataa ggaagaaaaa tgtagaattt tgatttttgg   1499

aaaaaaaaat aaagtttaat gtcagagatt gacagaaata agcatgtgag gcgaggccat   1559

atttgactat ttcaagatgt catagtaatc ctatttttct ttttcaaaaa taaaatgttg   1619

tctcaagatg tagtaccata tagaatatat taattaactt attatatatt acgtcatgga   1679

tgacaaattt gatgacgcca tcatagtgcc tatatttgtc cattaatcga cacataagca   1739

atgagtgcgt catcctagtg atgacgtcaa taccgttacg taagccgaca aaagtgatag   1799

gtgatgggtg gccttaactc atgattcggg tcgactcaaa atcggatttc tttattagaa   1859

taatttagaa taatttttat ttattaatta catcaatatt taatatttat tttatatcac   1919

aaatttaaaa aattattata ccttactaat atattatgtt taagtaaaaa atgttgcata   1979

aatatgaaga aatagtatta attgcctttc tttttttggc aagctctact gtttcttttt   2039

ttccttccta tttttttttt tttggtttaa agcaagtact gtttctattt gctacacaaa 2099

aagtactttt aacctcttta aatattcact cgctcgcttt ttatcgatcc aattatatgt 2159

taattattat attttgatac gaatttttct tttattataa aaagagatac ataataacct 2219

taaatcaaat aatttgttaa taactttaaa cgataataaa taaatatatt aataaaaaat 2279

gcttaaatat ttatcgacat tagttaaata tcgttaagtt taatatcgta aagcctttaa 2339

atacatattt gtcctctgaa attttttgat atagcaaata cataatttcc tttaaaatta 2399

aattcatttt aatccatccc agcccataaa tgacttatac tgcttttgac aatttcagac 2459

ccatcatttt cgttcttgtc gtacaatttc cccagttctt ttcattccca agtgtcaata 2519

tccaaaattt tatgattgga ctaatatata gttatattgc ataaatttat caaatttcca 2579

tattataagg cttgaatcta gacttttgaa tgtgggcata gatattttat accttctatc 2639

tacctcttgc tcaaaaagta aataaaagag tgctcaataa agaaattata taagtttttt 2699

taattgaaat ttgtagatct tttctttaaa gtgaagaata gtcgagaaac gaattaattt 2759

tctagtgtaa catgttgcgc tataattaag ccgaacaagg acaaacaaca ttaaataaac 2819

aagataaata taatattatt agttttatgt acccccataa caaagtgtct cgacccttga 2879

atgtgtaccc aaaaagtaac agtgcccttt gacaatctcg aatgttgaaa aatacaatta 2939

acacagcaaa tgaccatatt acccttggtt acgaagcaaa catgtcgggg aaaacctgct 2999

tctagacttc tctatatagg ttattcgtct aacaattatt gtccaataaa caagtagttt 3059

atgctctctc cgtttcattt tagatatcat cttttgaatt tgaagagtca aattagttta 3119

cgtttgatcg taaaattttc atagatcttt taaacatgtt aaattatcaa ttattatgac 3179

tcatagtact ttttacgtag tttacaaata tataaatttc attccaaaaa attaaagatt 3239

ttatgcgcaa atttttaatt aaaattaaac tcaaaacgta tagatgtcat aaatattgga 3299

acaaattgtt tatttttact cattatagac tcgtagcgat attttacata agtatttaat 3359

ttttctgttt gtatactaat gagtaatgat gacaattaag ttatatttta aacaaaaaaa 3419

atgaaggtgg gtaagcaatg aggatctgta acgtgaaaga taagaatatg ctgattttgt 3479

tgagtttcat ggctaaaaaa acgcgttttt tcaggattaa tttagcaaat gaaagtgact 3539

tgcaacataa attcaaatgg cataacgtaa gccaagccaa tgaataatgg ggcttttagt 3599

tcaattatag agcatttgat aaaataataa aaagtgagtg gagatgttga attagaaagc 3659

cagtagtgcc ttttttggat cccaactgat tacgcccaaa acactctcac tagattaggg 3719

tacataataa aatgcattaa tattcgaaga attaatgtaa atagtcgttg ttacag g 3776

gaa atc gtg agc aga tat tgc agg gaa att cgt caa ctc gga ttt aga 3824
Glu Ile Val Ser Arg Tyr Cys Arg Glu Ile Arg Gln Leu Gly Phe Arg
150                 155                 160                 165

tta gaa gaa gcc ata gca gaa agc ctg ggg tta gat aaa gag tgt ata 3872
Leu Glu Glu Ala Ile Ala Glu Ser Leu Gly Leu Asp Lys Glu Cys Ile
                170                 175                 180

aaa gat gta ttt ggt gaa caa gga caa cat atg gct atc aat tat tat 3920
Lys Asp Val Phe Gly Glu Gln Gly Gln His Met Ala Ile Asn Tyr Tyr
            185                 190                 195

cct cct tgt cca caa cca gaa ctt act tat ggg ctt ccg gcc cat act 3968
Pro Pro Cys Pro Gln Pro Glu Leu Thr Tyr Gly Leu Pro Ala His Thr
        200                 205                 210

gat cca aat tca ctt aca att ctt ctt caa gac ttg caa gtt gcg ggt 4016
Asp Pro Asn Ser Leu Thr Ile Leu Leu Gln Asp Leu Gln Val Ala Gly
    215                 220                 225

ctt caa gtt ctt aaa gat ggc aaa tgg tta gct gta aaa cct caa cct 4064
Leu Gln Val Leu Lys Asp Gly Lys Trp Leu Ala Val Lys Pro Gln Pro
230                 235                 240                 245

gac gcc ttt gtc att aat ctt ggg gat caa ttg cag gtaaaccaca 4110
Asp Ala Phe Val Ile Asn Leu Gly Asp Gln Leu Gln
                250                 255

actccttcaa atcgttataa gttaataata ataatcttta tgcaactcat tggagatatt 4170

aggtagtcgt tggattattg tatgaaattc gaatctaata gaaaattgtg attgtgcag 4229

gca gta agt aac ggt aag tac aga agt gta tgg cat cga gct att gtg 4277
Ala Val Ser Asn Gly Lys Tyr Arg Ser Val Trp His Arg Ala Ile Val
        260                 265                 270

aat tca gat caa gct agg atg tca gtg gct tcg ttt cta tgt ccg tgt 4325
Asn Ser Asp Gln Ala Arg Met Ser Val Ala Ser Phe Leu Cys Pro Cys
    275                 280                 285

gat agc gcg aaa atc agt gca cca aag ctg ctg aca gaa gat gga tct 4373
Asp Ser Ala Lys Ile Ser Ala Pro Lys Leu Leu Thr Glu Asp Gly Ser
290                 295                 300                 305

cca gtg att tat caa gac ttt acg tat gct gag tat tac aac aag ttc 4421
Pro Val Ile Tyr Gln Asp Phe Thr Tyr Ala Glu Tyr Tyr Asn Lys Phe
                310                 315                 320

tgg agc agg aat ttg gac cag caa cat tgt ttg gaa ctt ttc aag aac 4469
Trp Ser Arg Asn Leu Asp Gln Gln His Cys Leu Glu Leu Phe Lys Asn
            325                 330                 335

taa 4472

<210> 10
<211> 27
<212> DNA
<213> Lycopersicon esculentum

<400> 10
tcgaagaatt aatgtaaata gtcgttg 27

<210> 11
<211> 21
<212> DNA

<213>  Lycopersicon esculentum

<400>  11
cgatttgaag gagttgtggt t                                                21

## Description

### FIELD OF THE INVENTION

This invention relates to the field of plant biotechnology and plant breeding. In particular, the invention relates to a cultivated plant of the species *Solanum lycopersicum* that comprises a *dmr6* allele comprising one or more mutations on amino acid position 144 to 187, said mutations resulting in production of a mutant dmr6 protein that results in resistance against or tolerance for the fungal pathogen *Botrytis cinerea* compared to tomato plants that do not contain the mutant *dmr6* allele. The invention also relates to a fruit, a part of a fruit, a harvested fruit, a part of a harvested fruit, a seed, a seed coat, a leaf, a leaflet, a part of a leaf or a part of a leaflet, a cotyledon, a hypocotyl, a flower, a part of a flower, a root, a part of a root, a stem, a part of a stem, pollen, an ovary, an ovule, an oocyte, an anther, a cutting, a cell, a non-propagating cell, a tissue, an organ, progeny, a protoplast, a rootstock, a scion or a graft of the *Solanum lycopersicum* plants of the invention comprising said *dmr6* allele producing said protein. In addition, the invention provides methods for producing or reproducing said plant, mutated nucleic acids encoding a mutated dmr6 protein and a mutated dmr6 protein and a method for identifying plants of the invention.

### BACKGROUND OF THE INVENTION

*Solanum lycopersicum* plants are herbaceous plants of the family *Solanaceae,* originating in the New World. They have since become a mayor food crop. In 2012, FAOSTAT estimated world production at over 160 million tonnes. The species can tolerate most climates and does well in greenhouses, but is especially productive outdoors in warm climates. Single flowers bear the tomato fruit which is typically green when unripe, changing principally to red, although some varieties may remain green, or ripen to white, yellow, orange, pink, brown or purple. Tomatoes are susceptible to a number of pest and diseases, including insect and nematode pests, and bacterial, fungal, oomycete, viral, viroid and mycoplasma-like organisms [MLO] diseases.

Tomato breeding aims at the production of commercial varieties that have good agronomic properties, such as high yield, disease resistance, have desirable consumer characteristics, such as taste, especially sweetness. Use of disease resistant crops prevents crop loss, also allowing the growers to reduce use of crop protection compounds, which reduces costs and is desired by consumers and the communities where the crops are grown.

Fungal pathogens are a large threat to tomato yields; many of these fungi are *Ascomycota. Bottytis* is a genus of necrotropic fungi in the *Ascomycota* division which are an important pathological threat to over 200 plant species. The most important pathogenic fungus in the genus *Botrytis* is *Botrytis cinerea*, also spelled *Botrytis cynerea. Botrytis cinerea* causes a disease known as grey mould or gray mold, or colloquially Botrytis. One of the crops where *Botrytis cinerea* causes considerable damage and yield loss is tomato (O'Neil et al., 1997, Plant Disease, Vol 81 No 1 pp 36-40). *Botrytis cinerea* is known to spread easily in tomato greenhouses, and affects stems, leafs and fruits of tomato plants. Affected parts of plants wither, and affected fruits are unsalable. To reduce the probability of *Botrytis cinerea* infection, strict hygiene and careful regulation of the environment is required, but neither measure is fully effective. Non-organic growers may also use fungicides to prevent the spread of *Botrytis cinerea*, though some strains of *Botrytis cinerea* have become resistant against several commonly used fungicides (Leroux et al., 2002, Pest Management Science Vol 58 pp 876-888). As such, growers and consumers have an increasing demand for tomato varieties that are resistant to *Botrytis cinerea*, in order to reduce crop losses and/or use of fungicides. Genes conferring or enhancing *Botrytis cinerea* resistance are therefore continuously sought after by plant breeders in order to develop resistant (or less susceptible) cultivars.

Commercial tomato cultivars may contain a gene that confers resistance or tolerance against *Botrytis cinerea* or reduces susceptibility. Several sources of resistance and resistance genes are known in the art. Examples of known resistances are given in the following paragraphs.

WO2002085105 and WO2003090521 describe the identification of a Botrytis resistant donor plant selected from the group consisting of *Lycopersicon esculentum, Lycopersicon cerasiforme, Lycopersicon pimpinellifolium, Lycopersicon cheesmanii, Lycopersicon parviflorum, Lycopersicon chmielewskii, Lycopersicon hirsutum, Lycopersicon pennellii, Lycopersicon peruvianum, Lycopersicon chilense* and *Solanum lycopersicoides*; crossing the donor plant with a recipient plant and isolate genetic material from progeny plant; performing marker assisted selection with one or more molecular markers from chromosome 10 associated with at least one region on chromosome 10 that is linked to at least one gene that encodes for Botrytis resistance; and identifying those plants that contain DNA introgressed from the donor plant, where said introgressed DNA contains regions from chromosome 10 linked to at least one gene that encode for Botrytis resistance. All sources of the resistance conferring introgression on chromosome 10 are from wild relatives of tomato, in particular *L. hirsutum* (also known as *L. habrochaites*) accession LA1777, and require extensive work to transfer the resistance to cultivated material.

WO2006046861 describes two resistance sources, *Lycopersicon hirsutum (S. habrochaites*) LYC4/78 and *Lycopersicon parviflorum* G1.1601 that have "partial resistance to *Botrytis cinerea* in two lines of wild relatives of tomato. Furthermore, QTLs associated with the resistance were identified: on "chromosomes 1, 2 and 4 of *Lycopersicon hirsutum* LYC 4/78 and on chromosomes 3, 4 and 9 in *Lycopersicon parviflorum* G 1.1601. The resistance-levels are higher than those in WO2002085105 *(vide supra).* Both sources are accession lines from wild relatives of tomato, and require extensive work to transfer the resistance to cultivated material e.g. to remove linkage drag and/or other genetic material from the wild accession that has been crossed into the cultivated material.

WO2007123407 describes a resistance source "*Solanum habrochaites* LYC 4/78" that contain QTLs on "chromosome 4, 6, 9, 11 and/or 12" which are "involved in resistance to Botrytis". LYC 4/78 is the same accession as disclosed in WO2006046861.

WO201102079 describes a resistance source "*Solarium habrochaites* 04TEP990312" (accession P1 247087) that contains QTLs on at least one linkage group selected from linkage group 6, linkage group 1 b and linkage group 9b which are one genetic determinant directing or controlling expression of said resistance to Botrytis cinerea. WO2011020797 also uses a wild relative of cultivated tomato as a resistance conferring source.

Urbasch found 2 wild species *-L. columbianum* and *L. hirsutum-* to be resistant in each stage of development to 6 *B. cinerea*-isolates and additionally showed field resistance. Both sources are accession lines from wild relatives of tomato, and require extensive work to transfer the resistance to cultivated material (Urbasch 1986, J. Phytopathology, Vol 116 pp 344-351).

Egashira *et al* describe an assessment for resistance of the leaflet and stem in six tomato cultivars, 44 wild tomato accessions and a *Solanum lycopersicoides* accession. They found that *L. peruvianum* LA2745, *L. hirsutum* LA2314 and *L. pimpinellifolium* LA1246 showed high resistance both in the leaflet and in the stem, especially LA2745. Also these resistance-conferring sources are wild relatives of cultivated tomato (Egashira et al 2000, Biotic Stresses Vol 22 Issue 3 pp 324-326).

Nicot et al, 2002 discloses several dozen wild tomato accessions for *Botrytis cinerea* resistance, using measurement of stem and leaf lesions. Compared to *Solanum lycopersicum* these 20 accessions showed a reduction of symptoms, especially for *L. chmielewski* 731089 and *L. chilense* LA7969. Nicot et al did not report identification or introgression of any genetic determinant related to this resistance. Both sources are accession lines from wild relatives of tomato, and require extensive work to transfer the resistance to cultivated material (Nicot, P. et al, 2002 Report of the Tomato Genetics Cooperative Number 52 - September 2002 pp 24-26).

Finkers et al., 2007 disclose that partial resistance to *Botrytis cinerea* was identified in accessions of wild relatives of tomato such as *S. habrochaites* LYC4. They further disclose the identification of the loci "Rbcq1, a QTL reducing lesion growth (LG) and Rbcq2, a QTL reducing disease incidence (DI)" and "Rbcq4 on Chromosome 4". The source for the resistance is *S. habrochaites* LYC4.

Van Damme et al., 2005 disclose a gene *AtDMR6* in a *Arabidopsis* loss-of susceptibility screen to the pathogen *Hyaloperonospora parasitica.* The protein encoded by *AtDMR6* is an oxidoreductase. The truncated *A. thaliana* DMR6 mutant causes a sharp reduction in susceptibility to the oomycete pathogen *H. parasitica,* (a *Peronosporaceae* family pathogen causing Downy mildew in a.o. *Arabidopsis*) and reduces susceptibility but does not confer resistance to the ascomycete *Golovinomyces orontii* (a Erysiphaceae family pathogens causing Powdery mildew in *Arabidopsis*).The truncated mutant did not confer resistance to the bacterial pathogen *Pseudomonas syringae* pv. *tomato* in seedlings (Van Damme et al, 2005, Molecular Plant-Microbe Interactions, Vol 18, No 6 pp 583 - 592).

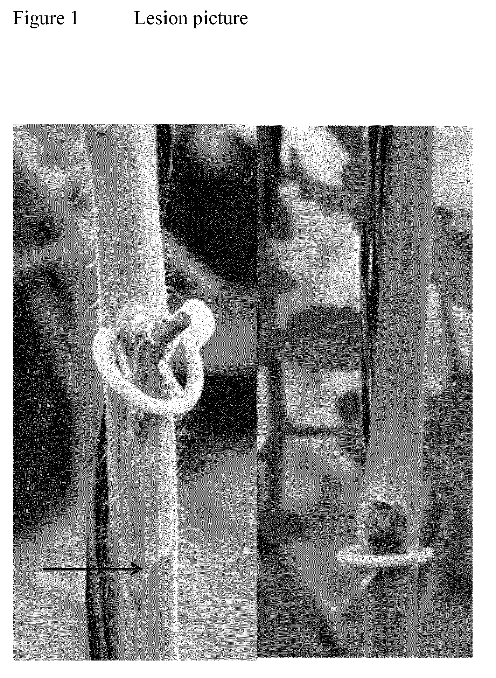

WO2008092505 discloses that lack of a functional AtDMR6 protein results in resistance against downy mildew (*Hyaloperonospora parasitica*), and that DMR6 functionality can be reduced by mutagenesis: WO2008092505 discloses that the resistance is based on an altered, in particular a reduced level, reduced activity or complete absence of the DMR6 protein *in planta.* It further discloses the mutants *Atdmr6-1*, which is truncated by a premature stop before the conserved catalytic domain (stop at position 141 - containing an incomplete DIOX-N domain) and *Atdmr6-2.* The application identifies two *Solanum lycopersicum* orthologs of AtDMR6 in figure 1 of the application, named Solanum lycopersicum_1 and Solanum lycopersicum_2. The application further describes that the reduced DMR6 function is suitable for for a large number of plant diseases caused by oomycetes such as, but not limited to, *Bremia lactucae* on lettuce, *Peronospora farinosa* on spinach, *Pseudoperonospora cubensis* on members of the *Cucurbitaceae* family, e.g. cucumber and melon, *Peronospora destructor* on onion, *Hyaloperonospora parasitica* on members of the *Brasicaceae* family, e.g. cabbage, *Plasmopara viticola* on grape, *Phytophthora infestans* on tomato and potato, and *Phytophthora sojae* on soybean. WO2008092505 does not disclose any experimental data of any resistance in tomato.

Van Damme et al 2008 describes an AtDMR6 mutant as a negative regulator of defense with a 2OG-Fe(ii) oxygenase domain, involved in the regulation of expression of other defense-related proteins. They further disclose that the *dmr6-2* mutant is a splicing mutant, inducing an 11-fold reduction in *wt* DMR6 protein level. The splicing mutant has a reduced susceptibility to *H. parasitica* i.e. nearly as strong as the truncation mutant (*dmr6-1*), but was not tested against *G*. *orontii* or *P. syringae* (Van Damme et al 2008 The Plant Journal Vol 54 pp 785-793).

Another study of AtDMR6 discloses that "*dmr6* (i.e. the truncation mutant) is also resistant to the bacterium *Pseudomonas syringae* and the oomycete *Phytophthora capsici*". Mature plants containing the truncation mutant of DMR6 exhibited higher resistance to *H. parasitica* than young plants, whereas plants overexpressing wt DMR6 were more susceptible to *H. parasitica* than parent lines. In the same screen, two mutants of the 2OG-Fe(ii) oxygenase catalytic centre, DMR6(H212Q) and DMR6(H269D), could not reinstate susceptibility of plants with the truncated DMR6 mutant (Zeilmaker et al, 2015, The Plant Journal Vol 81 pp 210 - 222).

Chapter 4 of Zeilmaker thesis discloses targeting residues in the catalytic centre of the 2OG-Fe(ii) oxygenase domain and in the substrate pocket of the DIOX-N domain for mutagenesis to adjust AtDMR6 function. This publication gives a proposed mechanism of action for DMR6, describing that DMR6 resistance is associated with enhanced defense gene expression and both resistance and defense were found to require salicylic acid and signalling through the key regulator NPR1, which signals downstream of salicylic acid. Furthermore, it states that DMR6 resistance requires a functional salicylic acid signalling pathway, that overexpression of DMR6 in *Arabidopsis* leads to enhanced susceptibility to *H. arabidopsidis* and *Pseudomonas syringae* pv *tomato,* and that DMR6 enzyme activity is required for plant disease susceptibility. In the paper, the three amino acids that compromise the catalytic triad that is essential for iron (FeII) binding and enzymatic activity" were mutated but the mutant versions corresponding to the substitutions H212Q, H269Q and H212Q_D214A, did not complement the resistance phenotype as transgenic plants remained resistant to *H. arabidopsidis.* The substrate pocket in the DMR6 model which contains six positively charged amino acids that are potentially involved in substrate binding was also investigated, replacing K105, R108, and R124 independently by alanine. It was found that DMR6 (K105A) is able to complement the *dmi-6-1* phenotype as plants became susceptible, indicating that the lysine at this position is not essential for DMR6 activity. On the other hand, plants expressing the DMR6 (R108A) and (R124A) substitutions are not able to complement the resistant phenotype when challenged with *H*. *arabidopsidis.* This indicates that these specific positions are crucial for DMR6 enzymatic function. This study exclusively focuses on the catalytic/substrate binding sites of the structured domains, and ignores all other parts of the protein (Tieme Zeilmaker, Thesis Utrecht University, Functional and applied aspects of the DOWNY MILDEW RESISTANT 1 and 6 genes in Arabidopsis, 2012, chapter 4).

WO2015193418 discloses *phytophthora* resistant plants belonging to the *Solanaceae* family. It discloses a plant belonging to the *Solanaceae* family wherein said plant comprises a genetic trait providing *Phytophthora* resistance and wherein said resistance trait is encoded by a combination of at least two genes having a reduced expression, or transcription, of said genes; or a reduced activity of proteins encoded by said genes as compared to said plant belonging to *Solanaceae* family being susceptible to *Phytophthora.* It especially relates to plants belonging to the *Solanaceae* family selected from the group consisting of sweet pepper *(Capsicum annuum* ), Capsicum spp., potato *(Solanum tuberosum*), petunia, tomato *(Solanum lycopersicum*), *Nicotinia benthamiana,* and tabacco (*Nicotinia tabacum*), and the present *Phytophthora* resistance is especially a resistance to a plant pathogen selected from the group consisting of *Phytophthora* spp., *Phytophthora capsici, Phytophthora infestans,* and *Phytophthora nicotinae.* Example 3 discloses transgenic RNAi silencing of two genes at the same time resulting in *Phytophthora* resistance in tomato.

Despite the above, there is a desire in the field for cultivated non-gmo tomato plants with a high resistance to *Botrytis.*

Resistance QTLs or genes introgressed from wild relatives of tomato often do not lead to high resistance, or may confer additional undesirable phenotypes such as linkage drag that cause low/sub-optimal agronomical performance. Regardless of origin, resistance QTLs or genes are regularly broken by pathogen strains, requiring new resistance sources.

It is an aim of the present invention to provide a new source for *Botrytis cinerea* resistance in cultivated tomato plants, and cultivated tomato plants that contain this new resistance against *Botrytis cinerea.* Preferably, the source of this new resistance against *Botrytis cinerea* is an endogenous gene of cultivated *Solanum lycopersicum*, instead of an introgression from a wild relative of tomato, for example an endogenous gene of cultivated *Solanum lycopersicum* comprising one or more mutations.

It is thus an object of the invention to generate and identify cultivated plants of the species *Solanum lycopersicum* that confer a reduced susceptibility or a resistance to fungi of the division *Ascomycota,* or more specifically of the family *Sclerotiniaceae*, or preferably that confer resistance or tolerance to Botrytis cinerea.

### SUMMARY OF THE INVENTION

The invention provides a cultivated plant of the species *Solanum lycopersicum* (i.e. cultivated tomato) comprising a *dmr6* allele encoding the DMR6 protein of SEQ ID No: 1 or a variant of SEQ ID No: 1 comprising at least 77% sequence identity to SEQ ID No: 1, wherein said DMR6 protein or variant comprises one or more mutations of an amino acid selected from amino acids 144 to 187 of SEQ ID No: 1 or the corresponding amino acids in said variant of SEQ ID No: 1. It is understood that SEQ ID No: 1 represents the wild type DMR6 protein sequence of tomato. Plants comprising such a *dmr6* allele show a reduced susceptibility or a resistance to fungi of the division *Ascomycota,* more specifically of the family *Sclerotiniaceae*, or even more specifically to *Botrytis cinerea.*

The invention further provides seeds from which such plants can be grown, and to parts of such plants. Methods relating to breeding with such plants or to propagation of such plants are also disclosed.

The invention further relates to a DMR6 protein of SEQ ID No: 1 or a variant thereof comprising one or more mutations at amino acid position 144 to 187 of SEQ ID No: 1 and to nucleic acid sequences encoding such proteins. Also the use of such proteins or nucleic acids for the identification of tomato plants comprising resistance or to identify resistance to a pathogenic fungus selected from the group of *Ascomycota, Sclerotiniaceae* and *Botrytis cinerea* in a *Solanum* plant or part thereof is part of the invention. Also methods to identify resistance to a pathogenic fungus selected from the group of *Ascomycota, Sclerotiniaceae* and *Botrytis cinerea* in a *Solanum lycopersicum* plant or part thereof comprising the step of identifying at least one of the above mentioned nucleic acids or mutant proteins is encompassed herein.

The invention also relates to the use of one or more mutations at amino acid position 144 to 187 of SEQ ID No: 1 or a variant thereof, to identify resistance to a pathogenic fungus selected from the group of *Ascomycota, Sclerotiniaceae* and *Botrytis cinerea* in a *Solanum* plant or part thereof. Likewise the invention relates to the use of a nucleic acid sequence encoding a protein comprising one or more mutations at amino acid position 144 to 187 of SEQ ID No: 1 or a variant thereof, to identify resistance to a pathogenic fungus selected from the group of *Ascomycota, Sclerotiniaceae* and *Bottytis cinerea* in a *Solanum* plant or part thereof.

### GENERAL DEFINITIONS

The verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one", e.g. "a plant" refers also to several cells plants, etc. Similarly, "a fruit" or "a plant" also refers to a plurality of fruits and plants. It is further understood that, when referring to "sequences" herein, generally the actual physical molecules with a certain sequence of subunits (e.g. amino acids) are referred to.

As used herein, the term "plant" includes the whole plant or any parts or derivatives thereof, preferably having the same genetic makeup as the plant from which it is obtained, such as plant organs (e.g. harvested or non-harvested fruits, leaves, flowers, anthers, etc.), plant cells, plant protoplasts, plant cell tissue cultures from which whole plants can be regenerated, plant calli, plant cell clumps, plant transplants, seedlings, plant cells that are intact in plants, plant clones or micropropagations, or parts of plants, such as plant cuttings, embryos, pollen, anthers, ovules, fruits (e.g. harvested tissues or organs), flowers, leaves, seeds, clonally propagated plants, roots, stems, root tips, grafts (scions and/or root stocks) and the like. Also any developmental stage is included, such as seedlings, cuttings prior or after rooting, etc. When "seeds of a plant" are referred to, these either refer to seeds from which the plant can be grown or to seeds produced on the plant, after self-fertilization or cross-fertilization. Seeds produced on the plant comprise both maternal tissues and tissues that result from fertilization.

"Plant variety" is a group of plants within the same botanical taxon of the lowest grade known, which (irrespective of whether the conditions for the recognition of plant breeder's rights are fulfilled or not) can be defined on the basis of the expression of characteristics that result from a certain genotype or a combination of genotypes, can be distinguished from any other group of plants by the expression of at least one of those characteristics, and can be regarded as an entity, because it can be multiplied without any change. Therefore, the term "plant variety" cannot be used to denote a group of plants, even if they are of the same kind, if they are all characterized by the presence of 1 locus or gene (or a series of phenotypically characteristics due to this single locus or gene), but which can otherwise differ from one another enormously as regards the other loci or genes.

"Plant part" refers to a part of a plant comprising a fruit or berry, a part of a fruit, a harvested fruit, a part of a harvested fruit, a seed, a seed coat, a leaf, a leaflet, a part of a leaf or a part of a leaflet, a cotyledon, a hypocotyl, a flower, a part of a flower, a root, a part of a root, a stem, a part of a stem, pollen, an ovary, an ovule, an oocyte, an anther, a cutting, a cell, a non-propagating cell, a tissue, an organ, progeny, a protoplast, a rootstock, a scion or a graft.

A "plant line" or "breeding line" refers to a plant and its progeny. As used herein, the term "inbred line" refers to a plant line which has been repeatedly selfed, e.g. 4, 5, 6, 7, or more times, and is homozygous for all or almost all loci.

"Tomato" refers herein to plants of the species *Solanum lycopersicum*, parts of plants and fruits or parts of fruits thereof. *Solanum lycopersicum* is also known as *Lycopersicon lycopersicum* (L.) H. Karst. or *Lycopersicon esculentum* Mill. The most commonly eaten part of a tomato is the fruit or berry.

"Cultivated plant" or "Cultivated tomato" refers to plants of *Solanum lycopersicum*, e.g. varieties, breeding lines or cultivars of the species *S. lycopersicum*, cultivated by humans and having good agronomic characteristics; preferably such plants are not "wild plants", i.e. plants which generally have much poorer yields and poorer agronomic characteristics than cultivated plants and e.g. grow naturally in wild populations. "Wild plants" include for example ecotypes, PI (Plant Introduction) lines, landraces or wild accessions or wild relatives of a species, or so-called heirloom varieties or cultivars, i.e. varieties or cultivars commonly grown during earlier periods in human history, but which are not used in modern agriculture.

"F1, F2, etc." refers to the consecutive related generations following a cross between two parent plants or parent lines. The plants grown from the seeds produced by crossing two plants or lines is called the F1 generation. Selfing the F1 plants results in the F2 generation, etc. "F1 hybrid" plant (or F1 seed) is the generation obtained from crossing two inbred parent lines. F1 hybrids are more vigorous and higher yielding, due to heterosis. Inbred lines are essentially homozygous at most loci in the genome. An "M1 population" is a plurality of mutagenized seeds / plants of a certain plant line or cultivar. "M2, M3, M4, etc." refers to the consecutive generations obtained following selfing of a first mutagenized seed / plant (M1).

The term "nucleic acid sequence" (or nucleic acid molecule) refers to a DNA or RNA molecule in single or double stranded form, particularly a DNA encoding a protein or protein fragment according to the invention. An "isolated nucleic acid sequence" refers to a nucleic acid sequence which is no longer in the natural environment from which it was isolated, e.g. the nucleic acid sequence in a bacterial host cell or in the plant nuclear or plastid genome.

The term "gene" means a DNA sequence comprising a region (transcribed region), which is transcribed into an RNA molecule (e.g. an mRNA, hpRNA or an RNAi molecule) in a cell, operably linked to suitable regulatory regions (e.g. a promoter). A gene may thus comprise several operably linked sequences, such as a promoter, a 5' leader sequence comprising e.g. sequences involved in translation initiation, a (protein) coding region (cDNA or genomic DNA [comprising exon and intron sequences]) and a 3' non-translated sequence comprising e.g. transcription termination sites. A gene may be an endogenous gene (in the species of origin) or a chimeric gene (e.g. a transgene or cis-gene).

A "mutation" in a nucleic acid molecule coding for a protein is a change of one or more nucleotides compared to the wild type sequence, e.g. by replacement, deletion or insertion of one or more nucleotides. A "point mutation" is the replacement of a single nucleotide, or the insertion or deletion of a single nucleotide. A "substitution in a nucleic acid molecule" is the replacement of a single nucleotide by a different single nucleotide. A "stop mutation" or "stop" is a mutation that results in a preliminary stop; the sequence following is not translated in a protein.

A "nonsense" mutation is a (point) mutation in a nucleic acid sequence encoding a protein, whereby a codon is changed into a stop codon. This results in a premature stop codon being present in the mRNA and in a truncated protein. A truncated protein may have reduced function or loss of function.

A "missense" or non-synonymous mutation is a (point) mutation in a nucleic acid sequence encoding a protein, whereby a codon is changed to code for a different amino acid. The resulting protein may have reduced function or loss of function.

A "splice-site" mutation is a mutation in a nucleic acid sequence encoding a protein, whereby RNA splicing of the pre-mRNA is changed, resulting in an mRNA having a different nucleotide sequence and a protein having a different amino acid sequence than the wild type. The resulting protein may have reduced function or loss of function.

A "frame-shift" mutation is a mutation in a nucleic acid sequence encoding a protein by which the reading frame of the mRNA is changed, resulting in a different amino acid sequence. The resulting protein may have reduced function or loss of function.

A mutation in a regulatory sequence, e.g. in a promoter of a gene, is a change of one or more nucleotides compared to the wild type sequence, e.g. by replacement, deletion or insertion of one or more nucleotides, leading for example to reduced or no mRNA transcript of the gene being made.

The term "allele(s)" means any of one or more alternative forms of a gene at a particular locus, all of which alleles relate to one trait or characteristic at a specific locus. In a diploid cell of an organism, alleles of a given gene are located at a specific location, or locus (loci plural) on a chromosome. One allele is present on each chromosome of the pair of homologous chromosomes. A diploid plant species may comprise a large number of different alleles at a particular locus. An individual diploid plant may contain identical alleles of the gene (homozygous) or two different alleles (heterozygous).

The terms "homologous" and "heterologous" refer to the relationship between a nucleic acid or amino acid sequence and its host cell or organism, especially in the context of transgenic organisms. A homologous sequence is thus naturally found in the host species (e. g. a tomato plant transformed with a tomato gene), while a heterologous sequence is not naturally found in the host cell (e. g. a tomato plant transformed with a sequence from potato plants). Depending on the context, the term "homolog" or "homologous" may alternatively refer to sequences which are descendent from a common ancestral sequence (e. g. they may be orthologs).

The term "locus" (plural loci) means a specific place or places or a site on a chromosome where for example a gene or genetic marker is found. The DMR6 locus is thus the location in the genome where the *DMR6* (wild type) or *dmr6* (mutant) gene is found.

When reference is made to a nucleic acid sequence (e.g. DNA, cDNA or genomic DNA) having "substantial sequence identity to" a reference sequence or having a sequence identity of at least 85%, e.g. at least 88%, 90%, 92%, 95%, 96%, 97%, 98% or more e.g. at least 99%, 99.2%, 99.3%, 99.4%, 99.5%, 99.6%, 99.7%, 99.8% or 99.9% nucleic acid sequence identity to a reference sequence, said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions.

"Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (T) for the specific sequences at a defined ionic strength and pH. The T is the temperature (under defined ionic strength and pH) at which 50% of the target sequence hybridises to a perfectly matched probe. Typically stringent conditions will be chosen in which the salt concentration is about 0. 02 molar at pH 7 and the temperature is at least 60°C. Lowering the salt concentration and/or increasing the temperature increases stringency. Stringent conditions for RNA-DNA hybridisations (Northern blots using a probe of e. g. 100 nt) are for example those which include at least one wash in 0. 2X SSC at 63°C for 20 min, or equivalent conditions. Stringent conditions for DNA-DNA hybridisation (Southern blots using a probe of e. g. 100 nt) are for example those which include at least one wash (usually 2) in 0. 2X SSC at a temperature of at least 50°C, usually about 55°C, for 20 min, or equivalent conditions. See also Sambrook et al. (1989) and Sambrook and Russell (2001).

Alternatively, a nucleic acid sequence (e.g. DNA, RNA, cDNA or genomic DNA) having "substantial sequence identity to" a reference sequence means a sequence which is identical to the reference sequence for all nucleotides except for 1, 2, 3, 4, 5, 6, 7, 8, 9, 10, 11, 12, 13, 14, 15, 16, 17, 18, 19, or 20 nucleotides.

Likewise a protein sequence having "substantial sequence identity to" a reference sequence means a protein sequence which is identical to the reference sequence for all amino acids except for 1, 2, 3, 4, 5, 6, 7, 8, 9, 10, 11, 12, 13, 14, 15, 16, 17, 18, 19, or 20 amino acids.

"Allelism test" refers to a genetic test whereby it can be tested whether a phenotype, e.g. botrytis resistance, seen in two plant lines or varieties is determined by the same gene or locus or by different genes or loci. For example, the plants to be tested are crossed with each other, the F1 is selfed and the segregation of the phenotypes amongst the F2 or further selfing or backcross progeny is determined. The ratio of segregation indicates if the genes or loci are allelic.

"Expression of a gene" refers to the process wherein a DNA region, which is operably linked to appropriate regulatory regions, particularly a promoter, is transcribed into an RNA, which is biologically active, i.e. which is capable of being translated into a biologically active protein or peptide (or active peptide fragment) or which is active itself (e.g. in posttranscriptional gene silencing or RNAi). The coding sequence may be in sense-orientation and encodes a desired, biologically active protein or peptide, or an active peptide fragment.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous and, where necessary to join two protein encoding regions, contiguous and in reading frame so as to produce a "chimeric protein". A "chimeric protein" or "hybrid protein" is a protein composed of various protein "domains" (or motifs) which is not found as such in nature but which are joined to form a functional protein, which displays the functionality of the joined domains. A chimeric protein may also be a fusion protein of two or more proteins occurring in nature.

The terms "protein" or "polypeptide" are used interchangeably and refer to molecules consisting of a chain of amino acids, without reference to a specific mode of action, size, 3-dimensional structure or origin. A "fragment" or "portion" of a DMR6 protein may thus still be referred to as a "protein". An "isolated protein" is used to refer to a protein which is no longer in its natural environment, for example in vitro or in a recombinant bacterial or plant host cell.

A "substitution in a protein" is the replacement of a single amino acid by a different single amino acid.

An "active protein" or "functional protein" is a protein which has protein activity as measurable in vitro, e.g. by an in vitro activity assay, and/or in vivo, e.g. by the phenotype conferred by the protein. A "wild type" protein is a functional protein comprising at least 77% amino acid sequence identity to SEQ ID NO: 1 (also referred to as variant of SEQ ID NO:1), or preferably 78%, 79%, 80%, 85%, 90%, 92%, 94%, 95%, 96%, 97%, 98%, 99%, or 99.3% or even more preferably 99.7%. Likewise, the wild type *DMR6* allele is the allele encoding said wild type protein or variant. A "mutant protein" is herein a protein comprising one or more mutations in the nucleic acid sequence encoding the protein, whereby the mutation results in (the mutant nucleic acid molecule encoding) a protein having an altered function, e.g. measurable in vivo, e.g. by the phenotype conferred by the mutant allele. "Mutant proteins" comprise "reduced-function" or "loss-of-function" proteins, as e.g. measurable in vivo, e.g. by the phenotype conferred by the mutant allele, or in a disease resistance test.

"Wild type allele" (WT) refers herein to a version of a gene encoding a fully functional protein (wild type protein). Such a sequence encoding a fully functional DMR6 protein is for example the wild type *DMR6* cDNA (mRNA) sequence depicted in SEQ ID NO: 4, or the genomic DNA depicted in SEQ ID NO: 5. The protein sequence encoded by this wild type *DMR6* mRNA has 337 amino acids and is depicted in SEQ ID NO: 1, which corresponds to NCBI reference sequence NP_001233840.2. Variants of wt DMR6 protein sequences are disclosed in the NCBI Reference Sequence ADN39436.1, which has leucine at position 198, substituting the phenylalanine at 198 of SEQ ID No: 1, but is otherwise identical to SEQ ID NO: 1. Further variants of wt DMR6 are disclosed in NCBI Reference Sequence: XP_004241427.1; (www.ncbi.nlm.nih.gov/protein/ 460391637) (based on the genome generated from cultivated tomato variety Heinz 1706) and in WO2008092505, referred to a *Solanum_lycopersicum_2.* which have an amino acid sequence identity: of 79% with SEQ ID NO: 1. Other DMR6 protein encoding alleles may exist in other cultivated *Solanum lycopersicum* plants. The protein of SEQ ID NO: 1 consists of 337 amino acids. Two conserved domains have been detected for the DMR6 protein; i.e. a DIOX-N domain (comprises deduced amino acids 38-143) and a 2OG-Fe(II) oxygenase domain (deduced amino acids 188-288). The DIOX-N domain, a highly conserved non-haem dioxygenase in morphine synthesis N-terminal domain, is typical for proteins with 2-oxoglutarate/Fe(II)-dependent dioxygenase activity, and the 2OG-Fe(II) oxygenase domain, which contains a catalytic center of the functional protein of SEQ ID NO: 1. Furthermore, the protein contains three regions without defined structure, i.e. a.a. ∼1-37, ∼144-187 and ∼289-337. These three regions have no known function. The fully functional DMR6 protein may comprise substantial sequence identity with SEQ ID NO: 1, i.e. at least about 77%, 78%, 79%, 80%, 85% 90%, 95%, 97%, 98%, 99%, 99.3% or 99.7% sequence identity with SEQ ID NO: 1. Such fully functional wild type DMR6 proteins are herein referred to as variants of SEQ ID NO: 1. Likewise the nucleotide sequences encoding such fully functional DMR6 proteins are referred to as variants of the cDNA sequence of wt S1 DMR6 SEQ ID NO: 4 or the genomic DNA sequence of wt S1 DMR6 SEQ ID NO: 5.

"Variant(s) of the WT allele of dmr6" comprise(s) amongst others, NCBI Reference Sequences ADN39436.1 and NP_001233840.2, which both encode for a leucine on position 198, substituting the phenylalanine of SEQ ID No: 1, and NCBI Reference Sequence: XP_004241427.1 which encodes for a DRM6 protein with 79% amino acid sequence identity to SEQ ID No: 1.

"*DMR6*" is a gene encoding an oxidoreductase enzyme called "DMR6". DMR6 has no crystallographic structure, but a prediction with a high degree of confidence is possible based on structures of related proteins. The DMR6 protein contains a conserved 2OG-Fe(ii) oxygenase domain on the C-terminal side and a DIOX-N domain N-terminally, which is also highly conserved in the 2OG-Fe(ii) oxygenase class of oxidoreductases. The two domains of DMR6 are separated by an undefined amino acid sequence 44 residues long from amino acid position 144 to 187. This part is designated "linker". A cDNA sequence for a wt *Solanum lycopersicum DMR6* gene is given in SEQ ID No: 4. A genomic DNA sequence for a wt *Solanum lycopersicum DMR6* gene is given in SEQ ID No: 5. It is understood that the genomic DNA comprises introns compared to the cDNA, which result in a different numbering of the nucleic acids. The exons (expressed regions) of the gDNA as given in SEQ ID NO:5 are nucleotides 1 to 196 (exon 1); 280 to 526 (exon 2), 3773 to 4095 (exon 3) and 4225 to 4472 (exon 4). A "variant of a *DMR6* gene" is a *DMR6* gene encoding a fully functional DMR6 protein that has at least 77%,or preferably 78%, 79%, 80%, 85%, 90%, 95%, 98%, 99%, 99.2%, 99.3%, 99.4%, 99.5%, 99.6%, or even 99.7% amino acid sequence identity to SEQ ID No: 1.

A "mutant R165K *Solanum lycopersicum* dmr6 protein" is a *Solanum lycopersicum* dmr6 protein encoded by SEQ ID No: 1 that has a substitution mutation on position 165, replacing the positively charged arginine (R) with the smaller, but also positively charged amino acid lysine (K). The mutant R165K *Solanum lycopersicum* dmr6 protein is encoded by SEQ ID NO: 2. A "variant of a mutant R165K *Solanum lycopersicum* dmr6 protein" is a mutant R165K *Solanum lycopersicum* dmr6 protein having at least 77%,e.g. at least 78%, 79%, 80%, 85%, 90%, 95%, 98%, 99%, 99 %, 99.3% or even 99.7% amino acid sequence identity to SEQ ID No: 1 and comprising a lysine (K) on position 165 or the corresponding amino acid position. The mutant R165K *Solanum lycopersicum* DMR6 protein is exemplary of the substitution mutants on amino acid position 144 to 187 offering resistance to *Botrytis cinerea.* It is noted that nucleotide sequence SEQ ID NO: 6 is cDNA, i.e. coding DNA sequences, and nucleotide sequence SEQ ID NO: 7 is genomic DNA, both sequences encoding the protein of SEQ ID NO: 2. Obviously, when reference is made to cDNA nucleotide sequences, it is understood that the cDNA is the coding region of the corresponding *Solanum lycopersicum* genomic *dmr6* sequence, which, however, additionally contains introns and therefore the nucleotides have different numbering. The location of the exons in this mutant are at the same location as in the wild type gDNA; i.e. nucleotides 1 to 196 (exon 1); 280 to 526 (exon 2), 3773 to 4095 (exon 3) and 4225 to 4472 (exon 4).

Further, when reference is made to a tomato plant comprising a nucleotide sequence encoding a protein according to the invention (e.g. the mutant protein of SEQ ID No: 2 or a variant thereof), this encompasses different nucleotide sequences, due to the degeneracy of the genetic code. In one embodiment the plant comprises the genomic *dmr6* sequence depicted in SEQ ID NO:7 or a genomic *dmr6* sequence substantially identical thereto (e.g. having at least about 80%, 85%, 90%, 95%, 98%, 99%, 99.2%, 99.3%, 99.4%, 99.5%, 99.6%, 99.7% sequence identity with SEQ ID NO: 7), but with one or more mutations in said sequence, especially in the exons of said genomic sequence coding for amino acid position 144 to 187 of the dmr6 protein.

A "mutant L185F *Solanum lycopersicum* dmr6 protein" or "mutant S1 dmr6 protein" is a *Solanum lycopersicum* dmr6 protein encoded by SEQ ID No: 1 that has a substitution mutation on position 185, replacing the small hydrophobic, aliphatic amino acid leucine (L) with the larger, but also hydrophobic neutral nonpolar amino acid phenylalanine (F). A "variant of a mutant L185F S1 dmr6 protein" is a mutant L185F S1 dmr6 protein having at least 77%, e.g. at least 78%, 79%, 80%, 85%, 90%, 95%, 98%, 99%, 99.2%, 99.3%, 99.4%, 99.5%, 99.6%, or even 99.7% amino acid sequence identity to SEQ ID No: 1 and having a phenylalanine (F) on position 185 or the corresponding amino acid position in the variant of SEQ ID No: 1. The mutant L185F Sldmr6 protein is encoded by SEQ ID No: 3. It is noted that nucleotide sequence SEQ ID No: 8 is cDNA, i.e. coding DNA sequences, and nucleotide sequence SEQ ID No: 9 is genomic DNA, both sequences encoding the protein of SEQ ID No: 3. Obviously, when reference is made to these cDNA nucleotide sequences, it is understood that the cDNA is the coding region of the corresponding *Solanum lycopersicum* genomic *dmr6* sequence, which, however, additionally contains introns and therefore the nucleotides have different numbering. The location of the exons in this mutant are at the same location as in the wild type gDNA; i.e. nucleotides 1 to 196 (exon 1); 280 to 526 (exon 2), 3773 to 4095 (exon 3) and 4225 to 4472 (exon 4).

Further, when reference is made to a tomato plant comprising a nucleotide sequence encoding a protein according to the invention (e.g. the mutant protein of SEQ ID No: 3 or a variant thereof), this encompasses different nucleotide sequences, due to the degeneracy of the genetic code. In one embodiment the plant of the invention comprises the genomic *dmr6* sequence depicted in SEQ ID No: 9 or a genomic *dmr6* sequence substantially identical thereto (e.g. having at least about 80%, 85%, 90%, 95%, 98%, 99%, 99.2%, 99.3%, 99.4%, 99.5%, 99.6%, 99.7% sequence identity with SEQ ID NO: 9), but with one or more mutations in said sequence, especially in the exons of said genomic sequence coding for amino acid position 144 to 187 of the dmr6 protein

The "linker" or " linker domain" is an amino acid sequence approximately 44 residues long. It is typically calculated to start at position 144 and ending at position 187 of SEQ ID No: 1, or the equivalent positions on a variant of SEQ ID No: 1 The linker has no known or BLAST-predicted structure. There are no known interactions between individual amino acids of the linker, interactions between (parts of) the linker and the other domains of DMR6, and possible quaternary interactions (e.g. interactions with other proteins, nucleic acids or small molecules) involving one or more domains of DMR6. The current inventors surprisingly found that one or more mutations of an amino acid in this "linker domain" are confers resistance to botrytis in tomato plants.

"Sequence identity" and "sequence similarity" can be determined by alignment of two peptide or two nucleotide sequences using global or local alignment algorithms. Sequences may then be referred to as "substantially identical" or "essentially similar" when they are optimally aligned by for example the programs GAP or BESTFIT or the Emboss program "Needle" (using default parameters, see below) share at least a certain minimal percentage of sequence identity (as defined further below). These programs use the Needleman and Wunsch global alignment algorithm to align two sequences over their entire length, maximizing the number of matches and minimises the number of gaps. Generally, the default parameters are used, with a gap creation penalty = 10 and gap extension penalty = 0.5 (both for nucleotide and protein alignments). For nucleotides the default scoring matrix used is DNAFULL and for proteins the default scoring matrix is Blosum62 (Henikoff & Henikoff, 1992, PNAS 89, 915-919). Sequence alignments and scores for percentage sequence identity may for example be determined using computer programs, such as EMBOSS (http://www.ebi.ac.uk/Tools/psa/emboss_needle/). Alternatively percent similarity or identity may be determined by searching against databases such as FASTA, BLAST, etc., but hits should be retrieved and aligned pairwise to compare sequence identity. Two proteins or two protein domains, or two nucleic acid sequences have "substantial sequence identity" if the percentage sequence identity is at least 77%, 78%, 79%, 80%, 85%, 90%, 95%, 98%, 99%, 99.3%, 99.7% or more (as determined by Emboss "needle" using default parameters, i.e. gap creation penalty = 10, gap extension penalty = 0.5, using scoring matrix DNAFULL for nucleic acids an Blosum62 for proteins). Such sequences are also referred to as "variants" herein, e.g. other variants of mutant *dmr6* alleles and mutant dmr6 proteins than the specific nucleic acid and protein sequences disclosed herein can be identified, which have the same effect on resistance to or tolerance for *Ascomycota* or *Sclerotiniaceae* or *Bottytis cinerea.*

A amino acid in a "corresponding position" is an amino acid that is a match in a pairwise alignment. The bioinformatics system used for "Sequence identity" and "sequence similarity" determination can be used for determining "corresponding position".

The "*Ascomycota*" form one of the divisions or phylums of the Fungi. They are also known as ascomycetes or "sac fungi". Species in this group of fungi encompass several important plant pathogens. *Sclerotiniaceae* is one of the families within *Ascomycota, Botrytis* a genus in this family and *Botrytis cinerea* a pathogenic species. The family of *Ascomycota* contains many pathogens that cause considerable damage to crops, including tomato.

*Ascomycota* are not *Oomycetes. Oomycetes* are no longer classified as fungi in current phylogenetic classification, in previous phylogenetic classifications *Oomycetes* were considered a different division or phylum from *Ascomycota.* Important plant diseases caused by *Ascomycota* include Tomato Fusarium Wilt (caused by *Fusarium oxysporum f. sp. lycopersici* and closely related species), leaf spot (caused by *Septoria*), and stem rot (*Sclerotinia*).

*"Botrytis cinerea*" is an ascomyte necrotrophic fungus, and a highly important plant pathogen. *Botrytis cinerea* is the name of the anamorph (non-sexual form), whereas the much rarer teleomorph (sexual form) is known as *Botryotinia fuckeliana* or *Botryotinia cinerea* It is understood by the skilled person that *Botrytis cinerea* may encompass reference to the common anamorph and the rare teleomorph. *Botrytis cinerea* first became known as a pathogen of grapes, but causes damage in some 200 other crops, including *Solanacea.* The plant disease caused by *Botrytis cinerea* is commonly called "gray mould", "grey mold" or "Botrytis", which will be used interchangeably. Symptoms of infection with *Botrytis cinerea* include soft rot, leaf spot, lesions, damping off, stem cankers and blights of flowers, fruits, stems, and foliage. *Botrytis cinerea* also causes postharvest rot of produce, such as tomato fruits in storage. The fungus first appears as white growth but soon darkens to gray, and exhibits a velvety structure. *Botrytis cinerea* can be recognized by the stout, dark brown or black, branching conidiophores and lighter coloured conidia which cover plant parts in severe infections, and by the arrangement of spores on branched conidiophores, which resembles grape clusters. If a leaf is infected, the fungus can grow into the stem and form lesions. Pruning scars may also be infected, resulting in lesions. In tomato, infections with *Botrytis cinerea* can reduce yield of tomato fruits by causing damage to the fruit thereby making said fruit unsaleable, damaging the plant so carries fewer or no fruits, or killing the plant. *Botrytis cinerea* is not subdivided in strains, since it is known to be very heterogeneous. The skilled person can easily isolate *Botrytis cinerea* from a field. Field isolates of *Botrytis cinerea* can vary significantly, but there is no known correlation to geographical regions.

"Resistance to *Botrytis cinerea*" or "*Botrytis* resistance" or "gray mould resistance" or "grey mold resistance" or " *Sclerotiniaceae* resistance" or " *Ascomycota r*esistance" refers to a restriction in growth and/or development of a specific pathogen (e.g. one or more strains) and/or a reduction in disease symptoms following infection with a specific pathogen (e.g. *Botrytis cinerea* or closely related *Botrytis* species or *Sclerotiniaceae* or *Ascomycota)* in plants comprising an effective (or functional) resistance gene relative to plants lacking an effective (or functional) resistance gene. Such a reduction in disease symptoms or reduction in growth and/or development of a pathogen can be determined using methods known in the art and / or methods shown elsewhere herein. It is understood that the experimental set-up of such a determination should be such that the results are statistically significant. A person skilled in the art will understand how to set up a measurement to statistically significantly determine if a plant has resistance compared to a wild type plant (lacking the mutant *dmr6* allele). For example the p-value of such a determination should be < 0.05.

A restriction in growth and/or development of a specific pathogen is herein defined as at least 10% less (e.g. at least 15%, 20%, 30%, 40%, 50%, 60%, 70%, 80%, 90%, 95% or even at least 99% less) symptoms caused by the pathogen to the plant, when compared to a tomato plant lacking such a resistance.

Resistance may be determined qualitatively (classifying plants of a particular line or variety as "resistant", "tolerant" or "susceptible") and/or resistance may be quantified, for example in resistance assays, by e.g. measured as percentage of plants of a particular line or variety surviving infection with *Botrytis cinerea* (% survival) or showing reduced, few or no systemic symptoms if only part of a plant is infected. In one embodiment a plant line or variety is said to be resistant towards *Botrytis cinerea* if it is qualified as "resistant" according to the symptoms, especially the systemic symptoms, that do not develop following infection or inoculation with *Botrytis cinerea,* which can be tested in a *Botrytis cinerea* resistance assay, e.g. as described herein. In one embodiment a plant line or variety is said to be resistant towards *Botrytis cinerea* if the plant line or variety shows essentially no systemic symptoms, i.e. if at least 60% of plants, preferably at least 70%,75%, 80%, 85%,90% or more (e.g. 92%,93%,94%,95%,98%,99% or 100%) show no symptoms. In one embodiment a plant line or variety is said to be resistant towards *Botrytis cinerea* if the plant line or variety shows essentially no systemic symptoms, i.e. if at least 60% of plants, preferably at least 70%,75%, 80%, 85%,90% or more (e.g. 92%,93%,94%,95%,98%,99% or 100%) show no systemic symptoms. In another embodiment, a plant line or variety is said to be resistant towards *Botrytis cinerea* if the plant line shows less development of symptoms of *Botrytis cinerea* infection such as soft rot, leaf spot, lesions, damping off, stem cankers and blights of flowers, fruits, stems, and foliage. In yet another embodiment, a plant line or variety is said to be resistant towards *Botrytis cinerea* if the plant line shows a lower degree of development of said symptoms.

In one embodiment, a *Botrytis cinerea* resistance test is done in the open field, preferably in a place where the disease occurs frequently. In an alternative embodiment a *Botrytis cinerea* resistance test is done in under protective covering, i.e. in a greenhouse, glasshouse, nethouse or under a tunnel. In another embodiment, a *Botrytis cinerea* resistance test is done *in vitro*, for example on isolated plant parts, such as leaves, stems and fruit. Furthermore, such a test can be done on plants or plant parts in various stages of development, for example once the cotyledon is developed, or at 1, 2, 3, 4, 5, 6, 7, 8, 9, 10, 11, 12, 13, 14, 15, 16, 17, 18, 19, 20, 22, 23, 24, 25, 26, 27, 28, 29, or 30 weeks. Resistance assays can be done in locations of high natural occurrence of *Botrytis* or, preferably, by inoculation with the pathogen. The skilled person is aware of various methods for inoculating a plant with Botrytis, for example application of liquids containing spore suspension to leaves (Imada et al., 2015), application of plugs of agar containing micellium of *B. cinerea* to leaves (Martinez-Hidalgo Front Microbiol. 2015) or application of a suspension of conidia to 4 weeks old plants (Flors et al., 2007) application of plugs of PDA media containing actively growing mycelia of *B. cinerea* to leaves (Martinez-Hidalgo Front Microbiol. 2015), or injuring/damaging plant tissue and then applying pathogens to the injured tissue, or injuring a plant part with an object that is inoculated with *Botrytis cinerea,* optionally letting the object stay in.

*Botrytis cinerea* resistance can be evaluated at various time points after infection, such as 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days or 28 days, or more days. The skilled person will understand that at least 3 plants, or preferably 4, 5, 6, 7, 8, 9, 10, 12, 15 or 20 or even more plants per line or variety should be tested under the same test conditions to evaluate whether a line or variety is resistant and that suitable control plants should be included.

"High resistance to *Botrytis cinerea*" or "complete resistance to *Botrytis cinerea*" indicates that the plant shows almost no (e.g. less than 10% of the plant or fruit is affected) or no symptoms (e.g. less than 5% of the plant or fruit is affected) of infection, respectively.

Resistance against *Botrytis cinerea* can be measured by various methods known in the art, for example on whole plants or on plant parts, such as leaves or fruits.

If a measurement is performed on the whole plants, incidence and measurement of lesion size, i.e. length or diameter after inoculation with the pathogen are common methods. Inoculation can be achieved by various methods, including application of liquids containing spore suspension to leaves (Imada et al., 2015), application of plugs of agar containing micellium of *B. cinerea* to leaves (Martinez-Hidalgo Front Microbiol. 2015) or application of a suspension of conidia to 4 weeks old plants (Flors et al., 2007). Inoculation with a conidial suspension (1,000, 000 conidia/ml) of *Botrytis cinerea* may be repeated on leaves and stems (WO2003090521). Time points for lesions measurement vary between 48 hours (Martínez-Hidalgo Front Microbiol. 2015) (Flors et al., 2007), three days (Imada et al., 2015), but can also be shorter or longer, depending on climate conditions and individual susceptibility.

If measurement is performed on leaves, inoculation is generally achieved by application of a solution of spores or conidia to the leaf (WO2006046861). Incidence of lesions can be recorded (WO2006046861 and/or disease damage level can be scored in classes by extend of necrosis after application of plugs of PDA media containing actively growing mycelia of B. cinerea to leaves (Martínez-Hidalgo Front Microbiol. 2015) after 72 h, or lesion diameter can be measured 72 h after application of a suspension of conidia to leaves from 12 weeks old plants (Flors et al., 2007). Stems could also be tested for disease incidence (WO2006046861).

Measurements on fruits can for example be performed by wounding and spray. In wounding, punctures of the fruit were inoculated with a suspension of conidia and scored for symptoms after 96 h (Flors et al., 2007). In spraying, fruit was sprayed with a suspension of conidia and scored for symptoms after 1- 3 weeks (Flors et al., 2007).

"Grey mould" or "gray mold" or "Botrytis" refers to the disease in *Solanum lycopersicum* that is caused by *Bottytis cinerea.*

A "lesion" is a zone of infected and/or pathogen-damaged tissue. Lesions are typically discoloured and contain tissue exhibiting pathogen-caused necrosis. Lesions can be caused by infection with fungi, including *Ascomycota* or, fungi of the family *Sclerotiniaceae*, or preferably, to *Botrytis* or, most preferably, to *Botrytis cinerea.* The infected tissues in the lesion have impaired function. Lesions can be distinguished from other kinds of damage, such as discolouration due to mechanical damage or scarring, by waiting for development of sporulation. Lesions can occur on various plant parts and in various stages of plant development. "Stem lesions" are lesions that occur on the stem of the plants. They commonly start from an infected leaf, a dead leaf, a leaf scar or stem damage, in particular stem damage caused by insects as well as mechanical or chemical damage. Stem lesions can encompass part of the circumference of the stem or, in rare cases, the whole circumference of the stem. If the whole circumference of the stem is affected, the plant parts growing from the affected stem will die, if it is the main stem the whole plant will die. A lesion that circumferences a stem indicates a severe infection. Lesions that encompass part of the circumference of the stem can be quantified by size. Lesion size can be measured as length along the stem, measured from furthest point to furthest point, or as surface damaged. Furthermore, lesions can be quantified by speed of development, severity of symptoms and presence of mould. A typical example of a lesion caused by *Botrytis cinerea* is shown in Figure 1. Lesions on immature green fruit can be recognized when they discolour to light brown or white, generally starting at the point of contact with infection. The fruit may develop soft rot where the inner tissue becomes mushy and watery while the fruit skin remains intact. Eventually, a grey fuzzy mold may develop and sclerotia may appear with fruit falling off the plant. Green fruit can also become infected directly by airborne spores instead of by contact with other infections. Lesions caused by infection with fungal pathogens can be distinguished from other kinds of damage, such as discolouration or scarring, by waiting for development of sporulation. The skilled person is able to distinguish between a lesion caused by *Botrytis cinerea* and a lesion caused by another fungus, by the brown or black, branching conidiophores and lighter coloured conidia and by the grape-cluster-like arrangement of spores on conidiophores.

"Average" refers herein to the arithmetic mean.

The term "food" is any substance consumed to provide nutritional support for the body. It is usually of plant or animal origin, and contains essential nutrients, such as carbohydrates, fats, proteins, vitamins, or minerals. The substance is ingested by an organism and assimilated by the organism's cells in an effort to produce energy, maintain life, or stimulate growth. The term food includes both substance consumed to provide nutritional support for the human and animal body.

### BRIEF DESCRIPTION OF THE FIGURES

Figure 1 Lesion picture: On the left site, a typical lesion is shown, formed 14 days after inoculation with *Botrytis cinerea* on a susceptible plant. On the left site, a typical lesion formed 14 days after inoculation with *Botrytis cinerea* on a resistant plant is shown. The lesion length can be measured from the infection point (i.e. at the ring) until the arrow. The right image shows a circular lesion around the infection point.

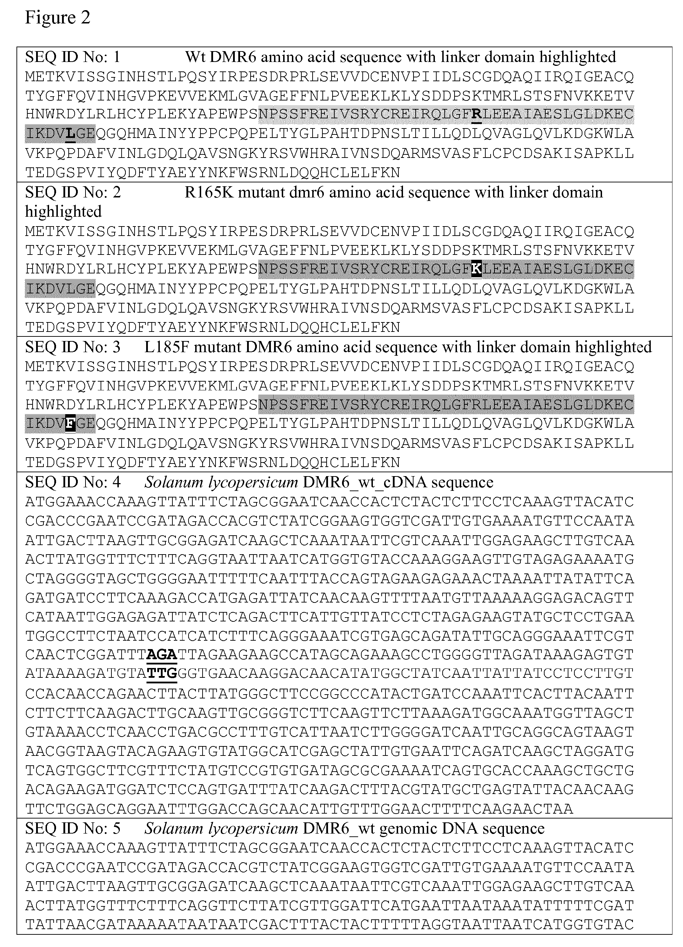

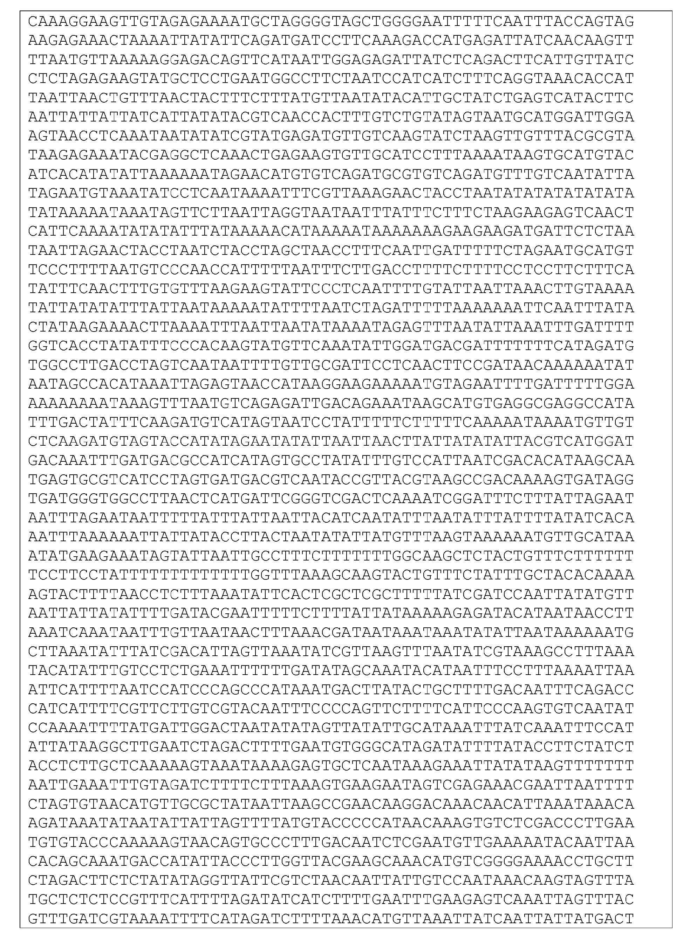

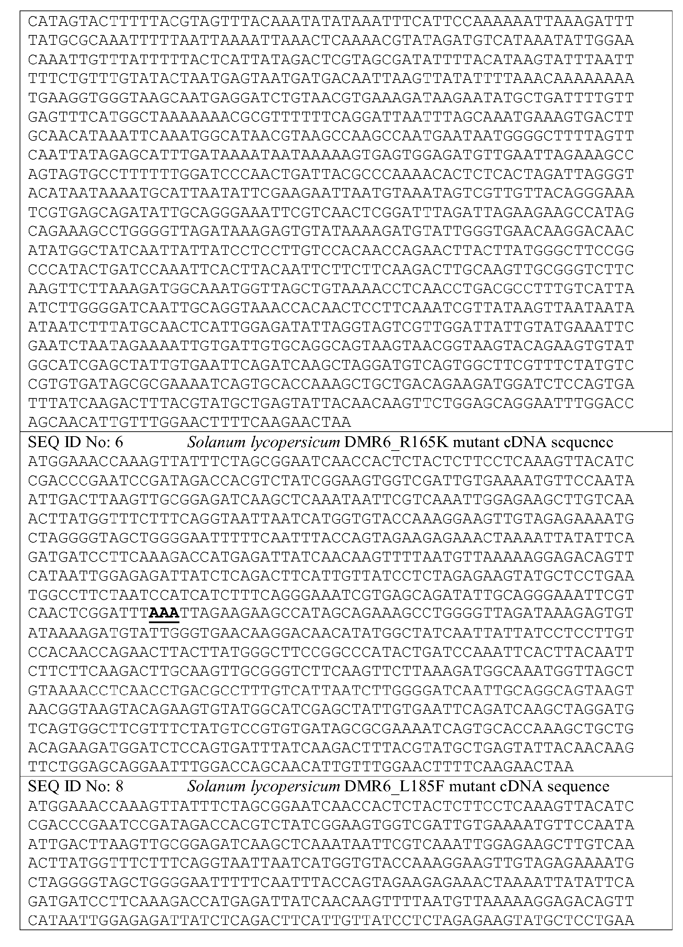

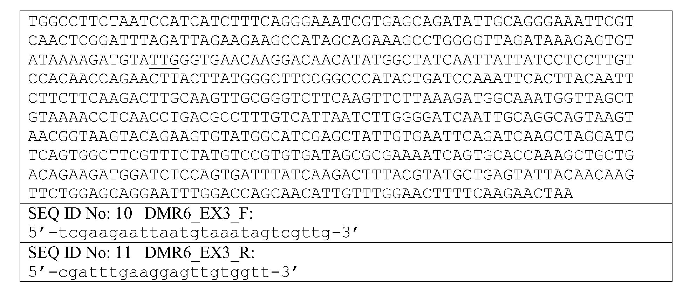

Figure 2 Sequences of amino acid sequence of wild type DMR6 protein sequence (SEQ ID NO: 1); R165K mutant protein sequence (SEQ ID NO: 2); L185F mutant protein sequence (SEQ ID NO: 3); as well as the cDNA encoding the wild type DMR6 protein sequence (SEQ ID NO: 4); the genomic DNA sequence encoding the wild type DMR6 protein sequence (SEQ ID NO: 5); the cDNA sequence encoding the R165K mutant (SEQ ID NO: 6); the cDNA sequence encoding the L185F mutant (SEQ ID NO: 8) and the primers SEQ ID NO: 10 (forward) and SEQ ID NO: 11 (reverse).

### BRIEF DESCRIPTION OF THE SEQUENCE LISTING

SEQ ID NO: 1 shows the *Solanum lycopersicum* wild type, fully functional, DMR6 protein sequence as derived from the mRNA generated from the Simcoe background. The sequence is identical to the sequence disclosed as "Solanum lycopersicum 1" in Figure 1 on WO2009092505. Amino acids 144-187 have been highlighted in gray. Amino Acids 165 and 185 have been highlighted.
SEQ ID NO: 2 shows a *Solanum lycopersicum* mutant dmr6 protein sequence as derived from the mRNA generated from a plant identified as having a mutant phenotype. The mutation involves a R165K amino acid substitution as compared to SEQ ID No: 1. Amino acids 144-187 have been highlighted in grey, and the substituted lysine amino acid is highlighted in white.
SEQ ID NO: 3 shows a *Solanum lycopersicum* mutant dmr6 protein sequence as derived from the mRNA generated from a plant identified as having a mutant phenotype. The mutation involves a L185F amino acid substitution as compared to SEQ ID No: 1. Amino acids 144-187 have been highlighted in grey, and the substituted phenylalanine amino acid is marked in white.
SEQ ID NO: 4 shows the *Solanum lycopersicum* wild type *DMR6* cDNA sequence as derived from the mRNA generated from the Simcoe background. The sequence is identical to the sequence disclosed as "Solanum lycopersicum 1" in Figure 1 on WO2009092505.
SEQ ID NO: 5 shows the *Solanum lycopersicum* wild type *DMR6* gDNA sequence as derived from the mRNA generated from the Simcoe background. The sequence is identical to the sequence disclosed as "Solanum lycopersicum 1" in Figure 1 on WO2009092505. The exons are located at nucleotides 1 to 196 (exon 1); 280 to 526 (exon 2), 3773 to 4095 (exon 3) and 4225 to 4472 (exon 4).
SEQ ID NO: 6 shows the *Solanum lycopersicum dmr6* R165K cDNA sequence as derived from the mRNA generated from the plant identified as having a mutant phenotype.
SEQ ID NO: 7 shows the *Solanum lycopersicum dmr6* R165K genomic DNA sequence as derived from the genome of the plant identified as having a mutant phenotype.
SEQ ID NO: 8 shows the *Solanum lycopersicum dmr6* L185F cDNA sequence as derived from the mRNA generated from the plant identified as having a mutant phenotype.
SEQ ID NO: 9 shows the *Solanum lycopersicum dmr6* L185F genomic DNA sequence as derived from the genome of the plant identified as having a mutant phenotype.
SEQ ID NO: 10 shows a first primer sequence used as forward primer
SEQ ID NO: 11 shows a second primer sequence (reverse primer)

### DETAILED DESCRIPTION OF THE INVENTION

This invention discloses a cultivated tomato, i.e. a cultivated plant of the species *Solanum lycopersicum*, comprising a *dmr6* allele (Downy Mildew Resistance 6, dmr6) encoding the DMR6 protein of SEQ ID No: 1, or a variant of SEQ ID No: 1 comprising at least 77% sequence identity to SEQ ID No: 1, wherein said DMR6 protein or variant comprises one or more mutations of an amino acid selected from amino acids 144 to 187 of SEQ ID No: 1 or the corresponding amino acids in said variant of SEQ ID No: 1 (i.e. the tomato plant of the invention comprising a *dmr6* allele encoding a mutant DMR6 protein ). It is understood that the numbering of specific amino acid residues in a protein sequence, such as SEQ ID No: 1, may change if one compares the sequence numbering of amino acids in a protein with the numbering in a variant of said protein. In one embodiment the DMR 6 protein or variant thereof comprises a mutation in any amino acid selected from the group consisting of amino acids 165 up to and including 185 of SEQ ID No: 1. In one embodiment the DMR 6 protein or variant thereof comprises a mutation in amino acid 165 or the corresponding position in the variant of SEQ ID No: 1. In another aspect, the DMR6 protein or variant thereof comprises a mutation in amino acid 185 or the corresponding position in the variant of SEQ ID No: 1.

In one aspect the plant of the invention does not comprise a truncated protein, i.e. amino acids 188 -337 of SEQ ID No: 1 are present in the mutant dmr6 protein.

In one aspect the invention discloses a cultivated tomato, comprising a *dmr6* allele encoding the DMR6 protein of SEQ ID No: 1 wherein said DMR6 protein comprises one or more mutations of an amino acid selected from amino acids 144 to 187 of SEQ ID No: 1.

In another aspect the invention discloses a cultivated tomato, comprising a *dmr6* allele encoding the DMR6 protein of a variant of SEQ ID No: 1 comprising at least 77% sequence identity to SEQ ID No: 1 e.g. 78%, 79%, 80%, 82%, 84%, 86, 87, 88, 89, 90, 91, 92, 93, 94, 95, 96, 97, 98, 98.3, 98.7, 99.0, or 99.3% or more preferably 99.7% sequence identity to SEQ ID NO: 1, (as determined using methods discloses elsewhere herein) wherein said DMR6 protein comprises one or more mutations of an amino acid selected from amino acids 144 to 187 of SEQ ID No: 1 or the corresponding position in a variant of SEQ ID No: 1. In yet another aspect, the invention discloses a cultivated tomato, comprising a *dmr6* allele encoding the DMR6 protein of a variant of SEQ ID No: 1 comprising at least 77% sequence identity to SEQ ID No: 1 e.g. 78%, 79%, 80%, 82%, 84%, 86, 87, 88, 89, 90, 91, 92, 93, 94, 95, 96, 97, 98, 98.3, 98.7, 99.0, or 99.3% or more preferably 99.7% sequence identity to SEQ ID NO: 1, (as determined using methods discloses elsewhere herein) wherein said DMR6 protein comprises one or more mutations of an amino acid selected from amino acids 165 or 185 of SEQ ID No: 1 or the corresponding position in a variant of SEQ ID No: 1. In one embodiment the mutation concerns a mutation of amino acid 165, in another embodiment, it concerns amino acid 185.

In still another aspect, the invention discloses a cultivated tomato, comprising a *dmr6* allele encoding the DMR6 protein of a variant of SEQ ID No: 1 comprising at least 77% sequence identity to SEQ ID No: 1 e.g. 78%, 79%, 80%, 82%, 84%, 86, 87, 88, 89, 90, 91, 92, 93, 94, 95, 96, 97, 98, 98.3, 98.7, 99.0, or 99.3% or more preferably 99.7% sequence identity to SEQ ID NO: 1, (as determined using methods discloses elsewhere herein) wherein said DMR6 protein comprises one or more mutations of an amino acid selected from amino acids 165 of SEQ ID No: 1 or the corresponding position in a variant of SEQ ID No: 1.

In one aspect the tomato plant of the invention comprising a *dmr6* allele encoding a mutant DMR6 protein comprising one or more mutations in the DMR6 protein of SEQ ID No: 1, comprises a single mutation. In one embodiment, this single mutation is at position 165 or at position 185 of SEQ ID No: 1. Such a mutation can be a substitution, a deletion, or an insertion. In one aspect, the mutation in the protein is a substitution, i.e. at least one amino acid of SEQ ID No: 1 is replaced by another amino acid.

In yet another aspect, the mutant DMR6 protein encoded by the tomato plant of the invention comprising a *dmr6* allele comprises a single mutation. In one embodiment, this single mutation is a substitution. In another aspect, this single mutation is a mutation of amino acid 165 or of 185 of SEQ ID No: 1. In still another embodiment, this single mutation is a substitution of amino acid 165 of SEQ ID No: 1, preferably a substitution from arginine to lysine (R165K). In a another aspect, this single mutation is a substitution of amino acid 185 of SEQ ID No: 1, preferably a substitution from leucine to phenylalanine (L185F).

In another aspect, the invention relates to a part of the plant of the invention (i.e. a cultivated tomato, comprising the *dmr6* allele encoding the mutant DMR6 protein). In one embodiment, said part is selected from the group consisting of a fruit, a part of a fruit, a seed, a seed coat, a leaf, a leaflet, a part of a leaf, a part of a leaflet, a cotyledon, a hypocotyl, a flower, a part of a flower, a root, a part of a root, a stem, a part of a stem, pollen, an ovary, an ovule, an oocyte, an anther, a cutting, a cell, a non-propagating cell, a tissue, an organ, progeny, a protoplast, a rootstock, a scion, and a graft.

In one embodiment, the mutant DMR6 protein, or the allele encoding such a mutant protein is still identifiable. In a further aspect, the invention relates to fruits of the plant of the invention. The invention also relates to seeds from which a plant of the invention can be grown.

Two specific mutant plants according to the invention (i.e. a cultivated tomato, comprising a *dmr6* allele encoding the DMR6 protein of SEQ ID No: 1 comprising at least 77% sequence identity with SEQ ID No: 1, wherein said DMR6 protein comprises one or more mutations of an amino acid selected from amino acids 144 to 187 of SEQ ID No: 1) have been identified. These specific mutant plants are exemplary for the current invention. The first plant, produces a mutant DMR6 protein comprising a substitution of amino acid 165 of SEQ ID No: 1, the second plant comprises a mutation of amino acid 185 of SEQ ID No: 1.

In one embodiment, the substitution on position 165 of SEQ ID NO: 1 is a substitution of the positively charged amino acid arginine with another polar amino acid selected from the group consisting of lysine (K), histidine (H), aspartic acid (D), glutamic acid (E), serine (S), threonine (T), asparagine (N) and glutamine (Q). In another aspect, the substitution on position 165 is a substitution of the positively charged amino acid arginine with another charged amino acid selected from the group of lysine (K), histidine (H), aspartic acid (D) and glutamic acid (E). In yet another aspect, the substitution on position 165 is a substitution of the positively charged amino acid arginine with another positively charged amino acid selected from the group of lysine (K) and histidine (H). Preferably, the substitution on position 165 is a substitution of the positively charged amino acid arginine with the smaller positively charged amino acid lysine (K).

In another aspect, the substitution on position 185 of SEQ ID NO: 1 is a substitution of the hydrophobic amino acid leucine with another hydrophobic amino acid selected from the group consisting of alanine (A), valine (V), isoleucine (I), methionine (M), phenylalanine (F), tyrosine (T) and tryptophan (W). In still another aspect, the substitution on position 185 is substitution of the hydrophobic amino acid leucine with another hydrophobic amino acid selected from the group consisting of isoleucine (I), phenylalanine (F), tyrosine (T) and tryptophan (W). Preferably, the substitution on position 185 is a substitution of the hydrophobic amino acid leucine with the hydrophobic amino acid phenylalanine.

It is understood that the multiplicity of three-codon combinations specifying an amino acid (i.e. the degeneracy of the genetic code), may cause different nucleotide sequences to encode the same protein. The codon for the amino acid at position 165 of SEQ ID No: 1 is AGA, which corresponds to nucleotides 493 - 495 of SEQ ID No: 6.

As stated above, in one embodiment the invention relates to the arginine at position 165 of SEQ ID No: 1 being substituted with a lysine. This is caused by a substitution of nucleotide 494 of SEQ ID No: 4 i.e. guanine, (G) with an adenine (A), as is depicted in SEQ ID No: 6. As a result the codon for amino acid 165 of SEQ ID No: 1 is changed from AGA (position 493-495 of SEQ ID No: 4) into AAA (position 493 - 495 of SEQ ID No: 6). The genomic DNA for this mutant (SEQ ID No: 7) differs from the wild type genomic DNA (SEQ ID No: 5) at position 3823: SEQ ID No: 7 has an adenine (A) at position 3823 while SEQ ID No: 5 has a guanine (G) at 3823.

Another embodiment of the invention relates to the leucine at position 185 of SEQ ID No: 1 being substituted with a phenylalanine. This is caused by a substitution of nucleotide 555 of SEQ ID No: 4: the guanine (G) is replaced with a thymine (T) as is depicted in SEQ ID No: 8. As a result the codon for amino acid 185 of SEQ ID No: 1 is changed from TTG (position 553 - 555) of SEQ ID No: 4 into TTT, respectively, as in SEQ ID No: 8. The genomic DNA for this mutant (SEQ ID No: 9) differs from the wild type genomic DNA (SEQ ID No: 5) at position 3884: SEQ ID No: 9 has a thymine (T) at position 3884 while SEQ ID No: 5 has a guanine (G) at 3884.

In yet another aspect, the invention relates to a cultivated tomato plant, comprising said *dmr6* allele encoding the mutant DMR6 protein, wherein said *dmr6* allele comprises SEQ ID No: 7, or can be transcribed in a nucleic acid sequence comprising SEQ ID No: 6. In still another aspect, said *dmr6* allele hybridises under stringent hybridization conditions to SEQ ID No: 6 or SEQ ID No: 7. In yet another aspect, said *dmr6* allele comprises a nucleotide sequence identical to SEQ ID No: 6 for all but 20 nucleotides, or all but 19, 18, 17, 16, 15, 14, 13, 12, 11, 10, 9, 8, 7, 6, 5, 4, 3, 2, or 1 nucleotide(s); and comprises an adenine (A) at position 494 of SEQ ID No: 6. In another aspect, said *dmr6* allele comprises a nucleotide sequence identical to SEQ ID No: 7 for all but 50 nucleotides, or all but 45, 40, 35, 30, 23, 20, 19, 18, 17, 16, 15, 14, 13, 12, 11, 10, 9, 8, 7, 6, 5, 4, 3, 2, or 1 nucleotide(s); and comprises an adenine (A) at position 3823 of SEQ ID No: 7.

In yet another aspect, the invention relates to a cultivated tomato plant, comprising said *dmr6* allele encoding the mutant DMR6 protein, wherein said *dmr6* allele comprises SEQ ID No: 9, or can be transcribed in a nucleic acid sequence comprising SEQ ID No: 8. In still another aspect, said *dmr6* allele hybridises under stringent hybridization conditions to SEQ ID No: 8 or SEQ ID No: 9. In yet another aspect, said *dmr6* allele comprises a nucleotide sequence identical to SEQ ID No: 8 for all but 20 nucleotides, or all but 19, 18, 17, 16, 15, 14, 13, 12, 11, 10, 9, 8, 7, 6, 5, 4, 3, 2, or 1 nucleotide(s); and comprises a thymine (T) or cytosine (C) at position 555 of SEQ ID No: 8. In another aspect, said *dmr6* allele comprises a nucleotide sequence identical to SEQ ID No: 9 for all but 50 nucleotides, or all but 45, 40, 35, 30, 23, 20, 19, 18, 17, 16, 15, 14, 13, 12, 11, 10, 9, 8, 7, 6, 5, 4, 3, 2, or 1 nucleotide(s); and comprises a thymine (T) at position 3884 of SEQ ID No: 9.

It is understood that in these aspect, nucleotides are numbered counting "A" in the ATG start codon as number 1.

In another aspect, said mutant dmr6 protein or variant thereof confers resistance or tolerance to fungi of the division *Ascomycota* or, preferably, fungi of the family *Sclerotiniaceae*, or, even more preferably, to *Botrytis* or, most preferably, to *Botrytis cinerea* compared to *Solanum lycopersicum* plants which are homozygous for the wild type fully functional *DMR6* allele (*DMR6* / *DMR6*) (encoding a functional *DMR6* protein of SEQ ID NO: 1 or a functional variant of said sequence).

In another aspect the invention relates to a plant of the invention comprising a dmr6 protein or a variant thereof comprising the aforementioned mutation(s) wherein said mutation or mutations confer intermediate or high resistance or tolerance or lack of susceptibility to *Botrytis cinerea* compared to *Solanum lycopersicum* plants which are homozygous for the wild type fully functional *dmr6* allele (*dmr6* / *dmr6*) (encoding a functional DMR6 protein of SEQ ID NO: 1 or a functional variant of SEQ ID NO: 1). In another aspect, the mutation or mutations in the plant of the invention confer intermediate or high resistance or tolerance or lack of susceptibility to fungi of the division *Ascomycota* or, preferably, fungi of the family *Sclerotiniaceae*, or, even more preferably, to *Botrytis* compared to *Solanum lycopersicum* being homozygous for the wild type *DMR6* allele encoding the protein of SEQ ID NO: 1 or a wild type variant of SEQ ID NO: 1 having at least 85% amino acid sequence identity to SEQ ID NO:1 but which does not comprise the the aforementioned mutation(s).

In another aspect the invention relates to a plant of the invention wherein said mutant dmr6 protein comprises amino acids 1 to 347 of SEQ ID NO: 1 and wherein said mutant dmr6 protein has one or more amino acids changed between position 144 and position 187, i.e. a change on one or more of the amino acid positions 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186 or 187 of SEQ ID NO: 1; or in the corresponding amino acid positions of a variant of SEQ ID NO: 1 having at least 77% sequence identity to SEQ ID NO: 1 or preferably 78%, 79%, 80%, 85%, 86, 87, 88, 89, 90, 91, 92, 93, 94, 95, 96, 97, 98, 98.3, 98.7, 99.0, or 99.3% or more preferably 99.7% sequence identity to SEQ ID NO: 1.

In a another aspect, the invention relates to a cultivated tomato plant comprising the mutant *dmr6* allele as found in / derivable from / obtainable from (or derived from or obtained from) seed deposited under accession number NCIMB 42541 (encoding SEQ ID No: 2; i.e. the R165K mutation). Said plant may comprise the mutant allele of the deposited material in one or two copies, i.e. in homozygous or heterozygous form. In heterozygous form, the other allele may be a wild type *dmr6* allele or a variant *dmr6* allele, such as from any one of the other mutants provided herein, or any other mutant *dmr6* allele encoding for a loss-of-function DMR6 protein or reduced-function DMR6 protein as described herein. In heterozygous form, the other allele may, thus, be a reduced function or a loss-of-function *dmr6* allele.

In yet another aspect, the invention relates to a cultivated tomato plant comprising the mutant *dmr6* allele as found in / derivable from / obtainable from (or derived from or obtained from) seed deposited under accession number NCIMB _ (encoding SEQ ID No: 3 i.e. the L185F mutation). Said plant may comprise the mutant allele of the deposited material in one or two copies, i.e. in homozygous or heterozygous form. In heterozygous form, the other allele may be a wild type *dmr6* allele or a variant *dmr6* allele, such as from any one of the other mutants provided herein, or any other mutant *dmr6* allele encoding for a loss-of-function DMR6 protein or reduced-function DMR6 protein as described herein. In heterozygous form, the other allele may, thus, be a reduced function or a loss-of-function *dmr6* allele.

In one aspect the plant is homozygous for the allele encoding the mutant dmr6 protein.

In another aspect the plant is heterozygous for the allele encoding the mutant dmr6 protein.

In still another aspect, the plant of the invention is an F1 hybrid plant.

In a further aspect, the invention relates to a seed from which a plant of the invention can be grown.

In another aspect, the invention relates to a cultivated tomato, comprising a *dmr6* allele encoding the mutant DMR6 protein wherein the *dmr6* allele is an endogenous tomato (*Solanum lycopersicum*) allele.

In still another embodiment the mutant *dmr6* allele encoding the mutant dmr6 protein comprising one or more mutations of an amino acid selected from amino acids 144-187 is derived from and/or generated in a cultivated tomato (e.g. a breeding line, variety or heirloom variety), i.e. endogenous. An example of such a cultivated variety is Simcoe. Such a human-induced mutation may, for example, be induced using targeted mutagenesis as described in EP1963505.

In still another aspect, the invention relates to an *dmr6* allele endogenous to cultivated *Solanum lycopersicum*, encoding the mutant dmr6 protein comprising one or more mutations of an amino acid selected from the group consisting of amino acids 144-187 said protein comprising an amino acid sequence having 85% sequence identity or preferably 86, 87, 88, 89, 90, 91, 92, 93, 94, 95, 96, 97, 98, 98.3, 98.7, 99.0, or 99.3% or more preferably 99.7% sequence identity to SEQ. ID NO: 2 and comprising the lysine at position 165.

In another aspect, the invention relates to an *dmr6* allele endogenous to cultivated *Solanum lycopersicum*, encoding the mutant dmr6 protein comprising one or more mutations of an amino acid selected from the group consisting of amino acids 144-187 said protein comprising an amino acid sequence having 85% sequence identity or preferably 86, 87, 88, 89, 90, 91, 92, 93, 94, 95, 96, 97, 98, 98.3, 98.7, 99.0, or 99.3% or more preferably 99.7% sequence identity to SEQ. ID NO: 3 and comprising the phenylalanine at position 185.

In yet another aspect, the invention relates to a cultivated tomato plant or plant part of the invention comprising an endogenous *dmr6* allele encoding the DMR6 protein having 100% sequence identity to SEQ. ID NO: 2 or to SEQ. ID NO: 3.

The invention further relates to tomato seeds, plants and plant parts comprising an endogenous *dmr6* gene encoding cDNA ( or an mRNA molecule or a sequence that hybridizes to said mRNA molecule) having at least 85% sequence identity to SEQ. ID NO: 2 or SEQ. ID NO: 3 and comprising at least one non-transgenic mutation within said endogenous *dmr6* gene, wherein the mutation results in an amino acid substitution at position 165 or 185 of the wild type DMR6 protein, or the corresponding position in a variant of SEQ. ID NO: 2 or SEQ. ID NO: 3.

In still another aspect, the invention relates to tomato fruit, seeds, pollen, plant parts, and/or progeny of a plant of the invention. In yet another aspect the invention relates to fruit of the plant of the invention. In a further aspect, the invention relates to seeds from which a plant of the invention can be grown.

In one aspect, the invention relates to tomato plants and /or fruits having resistance to fungi of the division *Ascomycota.* In another aspect to fungi of the family *Sclerotiniaceae*, or to *Botrytis.* In still another aspect the plants of the invention confer resistance to *Botrytis cinerea* caused by a non-transgenic mutation in the nucleic acids coding for a.a. 144-187 of SEQ ID NO: 1 of at least one *dmr6* allele, as described elsewhere herein.

In one aspect cultivated tomato plants according to the invention do not show symptoms if they are infected with a pathogenic fungus of the *Ascomycota,* in another aspect they show reduced symptoms. In still another aspect fewer plants are affected (e.g. show lesions of die) by the disease or the plant develops symptoms later.

In another aspect tomato plants according to the invention do not show symptoms if they are infected with a pathogenic fungus of the *Sclerotiniaceae*, or they show reduced symptoms, or no or fewer plants die of the disease or the plant develops symptoms later.

In yet another aspect tomato plants according to the invention do not show symptoms if they are infected with a pathogenic fungus of the *Sclerotiniaceae*, or they show reduced symptoms, or no or fewer plants die of the disease or the plant develops symptoms later.

In one aspect cultivated tomato plants according to the invention do not show symptoms if they are infected with *Botrytis cinerea,* e.g. the plant does not show signs of soft rot, leaf spot, lesions, damping off, stem cankers nor blights of flowers, fruits, stems, and foliage. In another aspect, tomato plants according to the invention show reduced symptoms if they are infected with *Botrytis cinerea;* the plant shows fewer signs of soft rot, leaf spot, lesions, damping off, stem cankers and blights of flowers, fruits, stems, and foliage. In yet another aspect tomato fruits according to the invention do not show post-harvest rot in storage if they are infected with *Botrytis cinerea,* or in another embodiment, they show a reduced susceptibility towards post-harvest rot when infected with *Botrytis cinerea* compared to wild-type plants.

In a further aspect cultivated tomato plants according to the invention do not show symptoms if they are infected with a California field isolate of *Botrytis cinerea,* e.g. the plant does not show signs of soft rot, leaf spot, lesions, damping off, stem cankers nor of blights of flowers, fruits, stems, and foliage. In another aspect, tomato plants according to the invention show reduced symptoms if they are infected with a California field isolate of *Botrytis cinerea* e.g. the plant shows fewer signs of soft rot, leaf spot, lesions, damping off, stem cankers or of blights of flowers, fruits, stems, and foliage. In yet another aspect of the invention tomato fruits according to the invention do not show postharvest rot in storage if they are infected with a California field isolate of *Bottytis cinerea.*

In still another aspect cultivated tomato plants according to the invention, or fruits thereof, do not exhibit necrosis or exhibit reduced necrosis if they are infected with *Botrytis cinerea,* or, in another aspect, a California field isolate of *Botrytis cinerea.*

In one aspect of the invention sporulation does not develop on cultivated tomato plants according to the invention, or fruits thereof, or sporulation develops later or more slowly on said plants if they are infected with *Botrytis cinerea,* or in another embodiment, a California field isolate of *Botrytis cinerea,.* Sporulation may develop 1, 2, 3, 4, 5, 6, 7, 8, 9, 10, 11,12, 13, 14 days or even more days later on a plant of the invention.

In a different aspect a cultivated tomato plants according to the invention, or fruits thereof, do not exhibit lesions or said plants have a lower incidence of lesions if they are infected with *Botrytis cinerea* or, in another aspect, a California field isolate of *Botrytis cinerea,.*

In a different aspect cultivated tomato plants according to the invention do not exhibit lesion that circumference the stem or said plants have a lower incidence of lesions that circumference the stem if they are infected with *Botrytis cinerea* or, in another aspect a California field isolate of *Bottytis cinerea.*

In a particular aspect of the invention cultivated tomato plants according to the invention have a significantly reduced lesion size compared to non-resistant tomato plants when infected with *Botrytis cinerea* or, in one aspect a California field isolate of *Botrytis cinerea.* The average size of the lesions on a susceptible plant are at least 50%, e.g. at least 75%, 100%, 150%, 200%, 250%, 300%, 400%, 500%, 600%, 800%, 1000%, 1250%, or at least 1500% larger than the size of lesions on the plant of the invention. In one aspect, the average stem lesion length on a susceptible plant is at least 50%, or even at least 75%, 100%, 150%, 200%, 250%, 300%, 400%, 500%, 600%, 800%, 1000%, 1250%, or at least 1500% longer measured along the stem than the size of lesions on the plant of the invention. In a further aspect, the average stem lesion surface on a susceptible plant is at least 50% larger, e.g. at least 75%, 100%, 150%, 200%, 250%, 300%, 400%, 500%, 600%, 800%, 1000%, 1250%, or at least 1500% larger than the surface of lesions on the plant of the invention. In still another aspect, stem lesions on a plant of the invention circumference less than 5%, 10%, 15%, 20%, 30%, 40%, 50%, 60%, 70%, 80%, 90%, 95% or 98% or 99% of the whole circumference while lesions on the susceptible plants encompass the whole circumference of the stem.

In a further aspect of the invention, a cultivated tomato plant according to the invention has a significantly lower rate of plant death compared to non-resistant tomato plants e.g. at least 5% or even at least 10%, 15%, 20%, 30%, 40%, 50%, 60%, 70%, 80%, 90%, 95%, 98% or at least 99% fewer plants die of grey mould when infected with *Botrytis cinerea* or, in one aspect with California field isolate of *Botrytis cinerea,* ( e.g. at least 50% fewer plants die of grey mould when infected with *Botrytis cinerea* or, in one aspect with California field isolate of *Botrytis cinerea).*

In a further aspect of the invention, cultivated tomato plants according to the invention have a significantly reduced rate of fruit falling of plants compared to non-resistant tomato plants e.g. 5%, 10%, 15%, 20%, 30%, 40%, 50%, 60%, 70%, 80%, 90%, 95% or 98% or 99% fewer fruits fall off when infected with *Botrytis cinerea* or, in another aspect a California field isolate of *Botrytis cinerea.*

Reduced susceptibility or resistance to or tolerance for *Botrytis cinerea* can have the advantage that fewer or no cultivated tomato plants die of grey mould, and/or that plants suffer less damage, and/or that fewer fruits fall off an infected plants, and /or that fewer fruits are damaged. Thus, reduced susceptibility or resistance to or tolerance for *Botrytis cinerea* increases harvestable yield of tomato fruits.

In still another aspect of the invention cultivated tomato plants are provided that have the same or similar resistance to *Botrytis cinerea* as tomato plants of the invention, of which representative seeds were deposited by Nunhems B.V. and accepted for deposit on 9 February 2016 at the NCIMB Ltd. (Ferguson Building, Craibstone Estate, Bucksburn Aberdeen, Scotland AB21 9YA, UK) according to the Budapest Treaty, under the Expert Solution (EPC 2000, Rule 32(1)). Seeds were given the following deposit number: NCIMB 42541 *(Solanum lycopersicum* R150070 comprising the dmr6 R165K mutation) and NCIMB XXXX *(Solanum lycopersicum* R150070 comprising the dmr6 L185F mutation).

In yet another aspect, the invention relates to cultivated tomato plants comprising the mutant *dmr6* allele as present in, or as obtainable from, or as obtained from, or as derivable from, or as derived from seeds deposited under NCIMB 42541 (*Solanum lycopersicum* R150070 comprising the dmr6 R165K mutation).

In still another aspect, the invention relates to cultivated tomato plants comprising the mutant *dmr6* allele as present in, or as obtainable from, or as obtained from, or as derivable from, or as derived from seeds deposited under NCIMB XXXX (*Solanum lycopersicum* R150070 comprising the dmr6 L185F mutation).

In one embodiment F1 hybrid tomato plants, or fruits or seeds thereof (i.e. seeds from which F1 hybrid tomato plants can be grown) are provided, comprising at least one *dmr6* allele encoding a dmr6 protein comprising a mutation in a.a.144-187, preferably a substitution on amino acid position 165 or 185, or variants comprising corresponding mutations. F1 hybrid seeds are seeds harvested from a cross between two inbred tomato parent plants. Such a F1 hybrid seed may comprise one or two mutant *dmr6* alleles according to the invention, and may be grown into a plant. Such a F1 hybrid comprising two mutant *dmr6* alleles according to the invention may comprise two copies of the same *dmr6* allele or two different *dmr6* alleles according to the invention, and may be grown into a plant. Thus, in one embodiment a plant according to the invention is used as a parent plant to produce an F1 hybrid, which have reduced susceptibility or resistance to or tolerance for *Botrytis cinerea* compared to wild type *DMR6*/*DMR6* plants.

According to a further aspect the invention provides a cell culture or tissue culture of the cultivated tomato plant of the invention. The cell culture or tissue culture comprises re-generable cells. Such cells can be derived from a fruit, a part of a fruit, a harvested fruit, a part of a harvested fruit, a seed, a seed coat, a leaf, a leaflet, a part of a leaf or a part of a leaflet, a cotyledon, a hypocotyl, a flower, a part of a flower, a root, a part of a root, a stem, a part of a stem, pollen, an ovary, an ovule, an oocyte, an anther, a cutting, a tissue, an organ, progeny, a protoplast, a rootstock, a scion or a graft.

Seeds from which plants according to the invention can be grown are also provided, as well as packages or containers containing such seeds. Also a vegetative propagation of plants according to the invention is an aspect encompassed herein. Likewise, harvested fruits and fruit parts, either for fresh consumption or for processing (i.e. the mutant allele or protein can still be identified). Fruits may be graded, sized and/or packaged. Fruits may be sliced or diced or further processed.

In another aspect the invention relates to one or more cells of a cultivated tomato plant of the invention.

The invention also relates to food and/ or food products comprising or consisting of the fruit, or part of a fruit, of a tomato plant of the invention. As used herein, food refers to nutrients consumed by human or animal species. Obviously, the mutant *dmr6* allele and / or dmr6 protein can still be identified in the food and / or feed product.

The mutant *dmr6* allele or dmr6 protein of a plant of the invention can be used to identify plants of the invention or in breeding methods.

In one aspect, the invention relates to a method of producing a tomato plant of the invention comprising the steps of:
a) obtaining plant material from a tomato plant;
b) treating said plant material with a mutagen to create mutagenized plant material;
c) analysing said mutagenized plant material to identify a plant having at least one mutation in at least one *dmr6* allele having substantial sequence identity to SEQ ID NO: 5 or in a functional variant thereof.

In these method, the plant material of step a) is preferably selected from the group consisting of seeds, pollen, plant cells, or plant tissue of a tomato plant line or cultivar. Plant seeds being more preferred. In another aspect, the mutagen used in this method is ethyl methanesulfonate. In step b) and step c) the mutagenized plant material is preferably a mutant population, such as a tomato TILLING population.

The method may further comprise analyzing the susceptibility and/or resistance to *Botrytis cinerea* of the selected plant or progeny of the plant and selecting a plant of which the fruit have reduced susceptibility or resistance or tolerance for to *Botrytis cinerea.*

In one aspect of these methods, the mutation is selected from a mutation resulting in an amino acid substitution at position 165 or 185 of SEQ ID NO: 1 or of a part thereof.

Thus, in one aspect a method for producing a tomato plant conferring reduced susceptibility and/or resistance or tolerance for to *Botrytis cinerea* is provided comprising the steps of:
a) providing a tomato TILLING population,
b) screening said TILLING population for mutants in the *dmr6* gene, and
c) selecting from the mutant plants of b) those plants (or progeny of those plants) which have reduced susceptibility or resistance or tolerance for to *Botrytis cinerea* compared to wild type (*dmr6*/*dmr6*) fruits.

Mutant plants (M1) are preferably selfed one or more times to generate for example M2 populations or preferably M3 or M4 populations for phenotyping. In M2 populations the mutant allele is present in a ratio of 1 (homozygous for mutant allele) : 2 (heterozygous for mutant allele): 1 (homozygous for wild type allele).

In yet a further aspect the invention relates to a method for producing a hybrid *Solanum lycopersicum* plant, said method comprising:
(a) obtaining a first *Solanum lycopersicum* plant of the invention or from a seed from which a plant of the invention can be grown; and
(b) crossing said first *Solanum lycopersicum* plant with a second *Solanum lycopersicum* plant to obtain hybrid seeds,
wherein said hybrid *Solanum lycopersicum* seeds comprise an *dmr6* allele having one or more mutations wherein said mutations result in production of a mutant DMR6 protein having a R165K or a L185F substitution in SEQ ID NO: 1 or in a variant of SEQ ID NO: 1.

Plants and plant parts (e.g. fruits, cells, etc.) of the invention can be homozygous or heterozygous for the mutant *dmr6* allele.

Preferably the plants according to the invention, which comprise one or more mutant *dmr6* alleles, that encode a mutant DMR6 protein comprising one or more substitutions in a.a.144-187 of SEQ ID NO: 1, the mutation preferably a substitution on position 165 or 185, do not produce fewer fruits, smaller fruits or lower quality fruits than the wild type plants.

Other putative DMR6 genes/proteins can be identified *in silico*, e.g. by identifying nucleic acid or protein sequences in existing nucleic acid or protein database (e.g. GENBANK, SWISSPROT, TrEMBL) and using standard sequence analysis software, such as sequence similarity search tools (BLASTN, BLASTP, BLASTX, TBLAST, FASTA, etc.).

The mutant DMR6 protein comprising one or more mutations on amino acid position 144 to 187 can be tested as described herein, by determining the effect this mutant allele has on reducing susceptibility or conferring resistance or tolerance for to *Botrytis cinerea.* Plants comprising a nucleic acid sequence encoding such a mutant dmr6 protein and having a reduced susceptibility or resistance to or tolerance for *Botrytis cinerea* compared to *Solanum lycopersicum* homozygous for the wild type *dmr6* allele can for example be generated using e.g. mutagenesis and identified by TILLING, as known in the art. Also transgenic methods can be used to test *in vivo* functionality of a mutant *dmr6* allele encoding a mutant dmr6 protein. A mutant allele can be operably linked to a plant promoter and the chimeric gene can be introduced into a tomato plant by transformation. Regenerated plants (or progeny, e.g. obtained by selfing), can be tested for reduced susceptibility or resistance to or tolerance for *Botrytis cinerea.* For example a tomato plant comprising a non-functional *dmr6* allele can be transformed to test the functionality of the transgenic *dmr6* allele.

TILLING (Targeting Induced Local Lesions IN Genomes) is a general reverse genetics technique that uses traditional chemical mutagenesis methods to create libraries of mutagenized individuals that are later subjected to high throughput screens for the discovery of mutations. TILLING combines chemical mutagenesis with mutation screens of pooled PCR products, resulting in the isolation of missense and nonsense mutant alleles of the targeted genes. Thus, TILLING uses traditional chemical mutagenesis (e.g. EMS or MNU mutagenesis) or other mutagenesis methods (e.g. radiation such as UV) followed by high-throughput screening for mutations in specific target genes, such as *dmr6* according to the invention. S1 nucleases, such as CEL1 or ENDO1, are used to cleave heteroduplexes of mutant and wildtype target DNA and detection of cleavage products using e.g. electrophoresis such as a LI-COR gel analyzer system, see e.g. Henikoff et al. Plant Physiology 2004, 135: 630-636. TILLING has been applied in many plant species, such as tomato. (see http://tilling.ucdavis.edu/index.php/Tomato_Tilling ), rice (Till et al. 2007, BMC Plant Biol 7: 19), Arabidopsis (Till et al. 2006, Methods Mol Biol 323: 127-35),-Brassica, maize (Till et al. 2004, BMC Plant Biol 4: 12), etc.

In another aspect, the invention relates to a method to identify a tomato plant of the invention comprising the step of:
a) analysing plant material having at least one mutation in at least one *dmr6* allele having substantial sequence identity to SEQ ID NO: 5 or in a functional variant thereof.

In still another aspect the invention relates to a method for producing a cultivated *Solanum lycopersicum* plant, comprising the steps of
a. crossing a plant of the invention with a second *Solanum lycopersicum* plant to obtain a progeny seed;
b. optionally harvesting said progeny seed;
c. optionally selecting progeny seed comprising the dmr6 allele of anyone of claim 1-11; and
d. optionally growing the selected progeny of step (c).

In still another aspect, the invention relates to a method for reproducing the cultivated *Solanum lycopersicum* plant of the invention comprising the step of asexual propagation the plant of the invention. Asexual (or vegetative) reproduction methods are known in the art and encompass methods like vegetative reproduction, slip plants or cuttings.

In a further aspect, the invention relates to the use of one or more mutations at amino acid positions 144-187 of SEQ ID No: 1 or a variant thereof to identify resistance to a pathogenic fungus selected from the group of *Ascomycota, Sclerotiniaceae* and *Botrytis cinerea* in a *Solanum* plant or part thereof. In yet another aspect, the invention relates to the use of one or more mutations at amino acid positions165 or 185 of SEQ ID No: 1 or a variant thereof.

In still another embodiment, the invention relates to the use of a mutation at amino acid position 165 of SEQ ID No: 1 or a variant thereof to identify resistance to a pathogenic fungus selected from the group of *Ascomycota, Sclerotiniaceae* and *Bottytis cinerea* in a *Solanum* plant (e.g. tomato) or part thereof. In one embodiment, the mutation is a R165K mutation in SEQ ID No: 1. In another aspect, the mutation is at amino acid position 185 of SEQ ID No: 1 or a variant thereof. In one embodiment, the mutation is a L185F mutation in SEQ ID No: 1. In one embodiment, the pathogenic fungus is *Botrytis cinerea.* In another embodiment of these uses, the *Solanum* plant is a *Solanum lycopersicum* plant.

In one aspect, the invention relates to a DMR6 protein of SEQ ID No: 1 or a variant thereof, comprising at least 77% sequence identity to SEQ ID No: 1, (e.g. at least 78%, 79%, 80%, 85%, 90%, 95%, 97%, 98%, 99%, 99.3% or even at least 99.7% amino acid sequence identity to SEQ ID No: 1, wherein said DMR6 protein or variant thereof comprises one or more mutations at amino acid position 144 to 187 of SEQ ID No: 1 or the corresponding amino acid position in a variant of SEQ ID No: 1.

In another aspect the invention relates to the DMR6 protein of SEQ ID No: 2 or a variant thereof comprising at least 77% e.g. at least 78%, 79%, 80%, 85%, 90%, 95%, 97%, 98%, 99%, 99.3% or even at least 99.7% amino acid sequence to SEQ ID No: 2 and comprising a Lysine at position 165, or the corresponding position in a variant thereof. In one embodiment, the invention relates to a nucleic acid encoding such a protein. In still another embodiment, the invention relates to a nucleic acid comprising SEQ ID No: 6 or SEQ ID No: 7.

In another aspect the invention relates to the DMR6 protein of SEQ ID No: 3 or a variant thereof comprising at least 77% e.g. at least 78%, 79%, 80%, 85%, 90%, 95%, 97%, 98%, 99%, 99.3% or even at least 99.7% amino acid sequence to SEQ ID No: 3 and comprising a phenylalanine at position 185, or the corresponding position in a variant thereof. In one embodiment, the invention relates to a nucleic acid encoding such a protein. In still another embodiment, the invention relates to a nucleic acid comprising SEQ ID No: 8 or SEQ ID No: 9.

In yet another aspect, the invention relates to a method to identify resistance to a pathogenic fungus selected from the group of *Ascomycota, Sclerotiniaceae* and *Botrytis cinerea* in a *Solanum lycopersicum* plant or part thereof comprising the step of identifying at least one of the nucleic acids selected from the group consisting of SEQ ID No: 6, SEQ ID No: 7, SEQ ID No: 8, and SEQ ID No: 9.

In still another aspect the invention relates to a method to identify resistance to a pathogenic fungus selected from the group of *Ascomycota, Sclerotiniaceae* and *Botrytis cinerea* in a *Solanum lycopersicum* plant or part thereof comprising the step of identifying a nucleotide sequence encoding amino acids 144 - 188 of SEQ ID No: 2. In one embodiment, the invention relates to a method to identify resistance to a pathogenic fungus selected from the group of *Ascomycota, Sclerotiniaceae* and *Botrytis cinerea* in a *Solanum lycopersicum* plant or part thereof comprising the step of identifying a nucleotide sequence encoding at least 5 e.g. 6, 7, 8, 9,. 10 or more consecutive amino acids SEQ ID No: 2, including amino acid at position 165 of SEQ ID No: 2 (e.g. in case of 5 amino acids: amino acids 161-165 or 162-166 or 163-167, 164-168 or 165-169 of SEQ ID No: 2).

In still another aspect the invention relates to a method to identify resistance to a pathogenic fungus selected from the group of *Ascomycota, Sclerotiniaceae* and *Botrytis cinerea* in a *Solanum lycopersicum* plant or part thereof comprising the step of identifying a nucleotide sequence encoding amino acids 144 - 188 of SEQ ID No: 3. In one embodiment, the invention relates to a method to identify resistance to a pathogenic fungus selected from the group of *Ascomycota, Sclerotiniaceae* and *Botrytis cinerea* in a *Solanum lycopersicum* plant or part thereof comprising the step of identifying a nucleotide sequence encoding at least 5 e.g. 6, 7, 8, 9,. 10 or more consecutive amino acids SEQ ID No: 3, including amino acid at position 185 of SEQ ID No: 3 (e.g. in case of 5 amino acids: amino acids 181-185 or 182-186 or 183-187, 184-188 or 185-189 of SEQ ID No: 3).

A one aspect, the invention relates to a method to identify resistance to a pathogenic fungus selected from the group of *Ascomycota, Sclerotiniaceae* and *Botrytis cinerea* in a *Solanum lycopersicum* plant or part thereof comprising the step of identifying a mutant of the dmr6 protein of SEQ ID No: 1 encoding a lysine at position 165 and/or a phenylalanine at position 185 in said plant or part thereof. In one embodiment the aspect relates to identifying the mutant dmr6 protein of SEQ ID No: 2 or SEQ ID No: 3.

A method to select a plant comprising a mutant *dmr6* allele or mutant dmr6 protein may, in one embodiment, further comprise the step of selecting the plant or part thereof comprising said nucleic acid, and, optionally, growing fruits from said plant.

A method to identify a plant comprising resistance to a pathogenic fungus as disclosed herein may, in one embodiment, further comprise the step of selecting the plant or part thereof comprising said nucleic acid, and, optionally, growing fruits from said plant.

In one embodiment of the invention (cDNA or genomic) nucleic acid sequences encoding such mutant DMR6 proteins with a mutation in amino acid 144-187 of SEQ ID NO: 1 comprise one or more non-sense and/or missense mutations, e.g. transitions (replacement of purine with another purine (A ↔ G) or pyrimidine with another pyrimidine (C ↔ T)) or transversions (replacement of purine with pyrimidine, or *vice verse* (C/T ↔ A/G). In one embodiment the non-sense and/or missense mutation(s) is/are in the nucleotide sequence encoding any of the *dmr6* exons, or an essentially similar domain of a variant DMR6 protein, i.e. in a domain comprising at least 80%, 85%, 90%, 95%, 98%, 99% amino acid sequence identity to amino acids of SEQ ID NO: 1 or to a variant thereof.

Also provided are nucleic acid sequences (genomic DNA, cDNA, RNA) encoding a dmr6 protein of SEQ ID NO: 1 with a mutation in amino acid 144-187, preferably encoding a substitution at position 165 or 185. In one embodiment, a nucleic acid is provided encoding the protein of SEQ ID NO: 2 or SEQ ID NO: 3; or variants thereof as defined above. Due to the degeneracy of the genetic code various nucleic acid sequences may encode the same amino acid sequence. The nucleic acid sequences provided include naturally occurring, artificial or synthetic nucleic acid sequences. A nucleic acid sequence encoding DMR6 is provided for in SEQ ID NO: 4 (wild type cDNA) and SEQ ID No; 5 (wild type genomic DNA). The nucleic acid can be isolated.

Also included are variant nucleic acid sequences, such as nucleic acid sequences hybridizing to a nucleic acid encoding a dmr6 protein of SEQ ID NO: 1 with one or more mutations in a.a.144-187, preferably a substitution on position 165 or 185 or, more preferably SEQ ID NO: 2 or SEQ ID NO: 3, under stringent hybridisation conditions as defined. Variants of such nucleic acid sequences also include nucleic acid sequences which have a sequence identity to SEQ ID NO: 6, SEQ ID NO: 7 or SEQ ID NO: 8 or SEQ ID No: 9 of at least 50% or more, preferably at least 55%, 60%, 70%, 80%, 85%, 90%, 95%, 98%, 99% or more (as determined by Emboss "needle" using default parameters, i. e. gap creation penalty = 10, gap extension penalty = 0. 5, scoring matrix nwsgapdna). It is clear that many methods can be used to identify, synthesise or isolate variants or fragments of said nucleic acid sequences, such as nucleic acid hybridisation, PCR technology, in silico analysis and nucleic acid synthesis, and the like.

It is understood that when sequences are depicted as DNA sequences while RNA is referred to, the actual base sequence of the RNA molecule is identical with the difference that thymine (T) is replace by uracil (U). When referring herein to nucleotide sequences (e.g DNA or RNA) italics are used, e.g. *dmr6* allele, while when referring to proteins, no italics are used, e.g. DMR6 protein. Mutants are in small letters (e.g *dmr6* allele or dmr6 protein), while wild type / functional forms are in capital letters (*DMR6* allele or DMR6 protein).

Also provided are nucleic acid sequences (genomic DNA, cDNA, RNA) encoding mutant dmr6 proteins with a mutation in a.a.144-187 of SEQ ID No: 1, preferably a substitution on position 165 or 185 or variants thereof as described above, and plants and plant parts comprising such mutant sequences. Said variant nucleic acid sequences may have 1, 2, 3, 4, 5, 6, 7, 8, 9, 10, 11, 12, 13, 14, 15, 16, 17, 18,, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 substitutions.

It is clear that many methods can be used to identify, synthesize or isolate variants or fragments of *dmr6* nucleic acid sequences, such as nucleic acid hybridisation, PCR technology, *in silico* analysis and nucleic acid synthesis, and the like.

A plant of the invention can be used in a conventional plant breeding scheme to produce more plants with the same characteristics or to introduce the mutated *dmr6* allele into other plant lines or varieties of the same or related plant species.

In another embodiment, the invention involves use of one or more mutations at a.a. 144-187 of SEQ ID No: 1 to identify and/or select a *Solanum* plant or part thereof resistant to a pathogenic fungus selected from the group of *Ascomycota, Sclerotiniaceae* and *Botrytis cinerea.* Said *Solanum* plant may be any member of the *Solanacea,* or, prefereably a potato *(Solanum tuberosum*)*,* a tomato *(Solanum lycopersicum* or a wild relative thereof, including S. *cheesmanii, S. chilense, S. habrochaites, S. chmielevvskii, S. glandulosum, S. hirsutum, S. minutum, S. parviflorum, S. pennellii, S. peruvianum, S. peruvianum* var. *humifusum* and *S. pimpinellifolium*,), a pepper *(Capsicum annuum*) a tobacco plant (*Nicotiana tabacum*) or a aubergine or eggplant *(Solanum melongena).* More preferably, the *Solanum* plant is a cultivated tomato.

Furthermore, the invention involves use of a single mutation at a.a. 144-187 of SEQ ID No: 1 to identify and/or select a *Solanum* plant (defined as above) or part thereof resistant to a pathogenic fungus selected from the group of *Ascomycota, Sclerotiniaceae* and *Botrytis cinerea.*

Also, the invention involves use of a substitution at a.a. 144-187 of SEQ ID No: 1 to identify and/or select a *Solanum* plant (defined as above) or part thereof resistant to a pathogenic fungus selected from the group of *Ascomycota, Sclerotiniaceae* and *Botrytis cinerea.*

Also, the invention involves use of a substitution at a.a. 165-185 of SEQ ID No: 1 to identify and/or select a *Solanum* plant (defined as above) or part thereof resistant to a pathogenic fungus selected from the group of *Ascomycota, Sclerotiniaceae* and *Botrytis cinerea.*

Also, the invention involves use of a substitution at a.a. 165 or 185 of SEQ ID No: 1 to identify and/or select a *Solanum* plant (defined as above) or part thereof resistant to a pathogenic fungus selected from the group of *Ascomycota, Sclerotiniaceae* and *Botrytis cinerea.*

Also, the invention involves use of a R165K and/ or a L185F substitution in SEQ ID No: 1 to identify and/or select a *Solanum* plant or part thereof resistant to a pathogenic fungus selected from the group of *Ascomycota, Sclerotiniaceae* and *Botrytis cinerea.*

Also transgenic plants of the invention can be made using the mutant *drm6* nucleotide sequences encoding a dmr6 protein comprising a mutation in a.a.144-187, preferably a substitution on amino acid position 165 or 185, or variants comprising corresponding mutations, using known plant transformation and regeneration techniques in the art. An "elite event" can be selected, which is a transformation event having the chimeric gene inserted in a particular location in the genome, which results in good expression of the desired phenotype.

The plants of the invention as described above can be homozygous for the mutant *dmr6* allele a dmr6 protein comprising a mutation in a.a.144-187, preferably a substitution on amino acid position 165 or 185, or variants comprising corresponding mutations. To generate plants comprising the mutant allele in homozygous form, selfing can be used. The mutant *dmr6* allele according to the invention can be transferred to any other tomato plant by traditional breeding techniques, such as crossing, selfing, backcrossing, etc. Thus any type of tomato having reduced susceptibility or resistance to or tolerance for *Botrytis cinerea.* due to the presence of at least one mutant *dmr6* allele encoding a dmr6 protein comprising a mutation in a.a.144-187, preferably a substitution on amino acid position 165 or 185, or variants comprising corresponding mutations, can be generated. Any *S. lycopersicum* may be generated and/or identified having at least one mutant *dmr6* allele encoding a dmr6 protein comprising a mutation in a.a.144-187, preferably a substitution on amino acid position 165 or 185, or variants comprising corresponding mutations, in its genome. The tomato plant may, thus, be any cultivated tomato, any commercial variety, any breeding line or other, it may be determinate or indeterminate, open pollinated or hybrid, producing fruits of any colour, shape and size. The mutant allele generated and/or identified in a particular tomato plant may be easily transferred into any other tomato plant by breeding (crossing with a plant comprising the mutant allele and then selecting progeny comprising the mutant allele).

The presence or absence of a mutant *dmr6* allele according to the invention in any tomato plant or plant part and/or the inheritance of the allele to progeny plants can be determined phenotypically and/or using molecular tools (e.g. detecting the presence or absence of the *dmr6* nucleotide sequence or dmr6 protein using direct or indirect methods).

Also a method for transferring a mutant *dmr6* allele encoding a dmr6 protein comprising a mutation in a.a.144-187, preferably a substitution on amino acid position 165 or 185, or variants comprising corresponding mutations" to another plant is provided, comprising providing a plant comprising a mutant *dmr6* allele in its genome, whereby the plant comprising the mutant allele produce fruits that show reduced susceptibility or resistance to or tolerance for *Botrytis cinerea.* due compared to *Solanum lycopersicum* being homozygous for the wild type *DMR6* allele (as described above), crossing said plant with another plant and obtaining the seeds of said cross. Optionally plants obtained from these seeds may be further selfed and/or crossed and progeny selected comprising the mutant allele having reduced susceptibility or resistance to or tolerance for *Botrytis cinerea.* due to the presence of the mutant allele compared to plants comprising the wild type *Dmr6* allele.

As mentioned, it is understood that other mutagenesis and/or selection methods may equally be used to generate mutant plants according to the invention. Seeds may for example be radiated or chemically treated to generate mutant populations. Also direct gene sequencing of *dmr6* may be used to screen mutagenized plant populations for mutant alleles. For example KeyPoint screening is a sequence based method which can be used to identify plants comprising mutant *acs2* alleles (Rigola et al. PloS One, March 2009, Vol 4(3):e4761).

Any part of the plant, or of the progeny thereof, is provided, including a fruit, a part of a fruit, a harvested fruit, a part of a harvested fruit, a seed, a seed coat, a leaf, a leaflet, a part of a leaf or a part of a leaflet, a cotyledon, a hypocotyl, a flower, a part of a flower, a root, a part of a root, a stem, a part of a stem, pollen, an ovary, an ovule, an oocyte, an anther, a cutting, a cell, a non-propagating cell, a tissue, an organ, progeny, a protoplast, a rootstock, a scion or a graft etc. comprising a mutant *dmr6* allele according to the invention in the genome. Also plant cell cultures or plant tissue cultures comprising in their genome a mutant *dmr6* allele are provided. Preferably, the plant cell cultures or plant tissue cultures can be regenerated into whole plants comprising a mutant *dmr6* allele in its genome. Also double haploid plants (and seeds from which double haploid plants can be grown), generated by chromosome doubling of haploid cells comprising an *dmr6* mutant allele, and hybrid plants (and seeds from which hybrid plants can be grown) comprising a mutant *dmr6* allele in their genome are encompassed herein, whereby the double haploid plants and hybrid plants havingreduced susceptibility or resistance to or tolerance for *Botrytis cinerea.* due according to the invention.

The invention further relates to an endogenous dmr6 protein having the human-induced non-transgenic mutation R165K or L185F in SEQ ID NO: 1 or an endogenous *dmr6* allele encoding such protein.

In one aspect, seeds are packaged into small and/or large containers (e.g., bags, cartons, cans, etc.). The seeds may be pelleted prior to packing (to form pills or pellets) and/or treated with various compounds, such as seed coatings.

Seed pelleting can be combined with film coating (Halmer, P. 2000. Commercial seed treatment technology. In: Seed technology and its biological basis. Eds: Black, M. and Bewley, J. D., pages 257-286). Pelleting creates round or rounded shapes, which are easily sown with modern sowing machines. A pelleting mixture typically contains seeds and at least glue and filler material. The latter could be, for example, clay, mica, chalk or cellulose. In addition, certain additives can be included to improve particular properties of the pellet, e.g., a seed treatment formulation comprising at least one insecticidal, acaricidal, nematicidal or fungicidal compound can be added directly into the pelleting mixture or in separate layers. A seed treatment formulation can include one of these types of compounds only, a mixture of two or more of the same type of compounds or a mixture of one or more of the same type of compounds with at least one other insecticide, acaricide, nematicide or fungicide.

Formulations especially suitable for the application as a seed treatment can be added to the seed in the form of a film coating including also the possibility of using the coating in or on a pellet, as well as including the seed treatment formulation directly into the pellet mixture. Characteristically, a film coating is a uniform, dust-free, water permeable film, evenly covering the surface of all individual seeds (Halmer, P. 2000. Commercial seed treatment technology. In: Seed technology and its biological basis. Eds: Black, M. and Bewley, J. D., pages 257-286). Besides the formulation, the coating mixture generally also contains other ingredients such as water, glue (typically a polymer), filler materials, pigments and certain additives to improve particular properties of the coating. Several coatings can be combined on a single seed.

In addition, several combinations with film coating are possible: the film coating can be added on the outside of the pellet, in between two layers of pelleting material, and directly on the seed before the pelleting material is added. Also more than 1 film coating layer can be incorporated in a single pellet. A special type of pelleting is encrusting. This technique uses less filler material, and the result is a 'mini-pellet'.

Seeds may also be primed. Priming is a water-based process that is performed on seeds to increase uniformity of germination and emergence from the soil, and thus enhance vegetable stand establishment. Priming decreases the time span between the emergence of the first and the last seedlings. Methods how to prime lettuce seeds are well known in the art (see, e.g., Hill et al HortScience 42(6): 1436, 2007). Seeds may also be treated with any of the herbicides, insecticides or fungicides listed below.

### Fruits/Vegetables Herbicides

Atrazine, Bromacil, Diuron, Glyphosate, Linuron, Metribuzin, Simazine, Trifluralin, Fluazifop, Glufosinate, Halosulfuron Gowan, Paraquat, Propyzamide, Sethoxydim, Butafenacil, Halosulfuron, Indaziflam.

### Fruits/Vegetables Insecticides

Aldicarb , Bacillus thuriengiensis, Carbaryl, Carbofuran, Chlorpyrifos, Cypermethrin, Deltamethrin, Abamectin, Cyfluthrin/beta-cyfluthrin, Esfenvalerate, Lambda-cyhalothrin, Acequinocyl, Bifenazate, Methoxyfenozide, Novaluron, Chromafenozide, Thiacloprid, Dinotefuran, Fluacrypyrim, Spirodiclofen, Gamma-cyhalothrin, Spiromesifen, Spinosad, Rynaxypyr, Cyazypyr, Triflumuron,Spirotetramat, Imidacloprid, Flubendiamide, Thiodicarb, Metaflumizone, Sulfoxaflor, Cyflumetofen, Cyanopyrafen, Clothianidin, Thiamethoxam, Spinotoram, Thiodicarb, Flonicamid, Methiocarb, Emamectin-benzoate, Indoxacarb, Fenamiphos, Pyriproxifen, Fenbutatin-oxid.

### Fruits/Vegetables Fungicides

Ametoctradin, Azoxystrobin, Benthiavalicarb, Boscalid, Captan, Carbendazim, Chlorothalonil, Copper, Cyazofamid, Cyflufenamid, Cymoxanil, Cyproconazole, Cyprodinil, Difenoconazole, Dimetomorph, Dithianon, Fenamidone, Fenhexamid, Fluazinam, Fludioxonil, Fluopicolide, Fluopyram, Fluoxastrobin, Fluxapyroxad, Folpet, Fosetyl, Iprodione, Iprovalicarb, Isopyrazam, Kresoxim-methyl, Mancozeb, Mandipropamid, Metalaxyl/mefenoxam, Metiram, Metrafenone, Myclobutanil, Penconazole, Penthiopyrad, Picoxystrobin, Propamocarb, Propiconazole, Propineb, Proquinazid, Prothioconazole, Pyraclostrobin, Pyrimethanil, Quinoxyfen, Spiroxamine, Sulphur, Tebuconazole, Thiophanate-methyl, Trifloxystrobin.

The invention further relates to the following numbered embodiments:
E1 A plant cell of a cultivated plant of the species *Solanum lycopersicum* comprising a *dmr6* allele encoding the DMR6 protein of SEQ ID No: 1 or a variant of SEQ ID No: 1 comprising at least 77% sequence identity to SEQ ID No: 1, wherein said DMR6 protein or variant comprises one or more mutations of an amino acid selected from amino acids 144 to 187 of SEQ ID No: 1 or the corresponding amino acids in said variant of SEQ ID No: 1.
E2. The plant cell of E1, wherein said one or more mutations is a single mutation.
E3. The plant cell of any of the E1 or E2, wherein the mutation is a substitution.
E4. The plant cell of any of E1-E3, wherein said DMR6 protein or variant comprises a mutation at amino acid position 165 of SEQ ID No: 1 or at amino acid position 185 of SEQ ID No: 1, or the corresponding amino acid positions in a variant of SEQ ID No: 1.
E5. The plant cell of E 4, wherein said mutation at amino acid position 165 of SEQ ID No: 1 is an arginine to lysine substitution (R165K), or wherein said mutation at amino acid position 185 of SEQ ID No: 1 is a leucine to phenylalanine substitution (L185F).
E6. The plant cell of any of E1-E5, wherein said *dmr6* allele is the allele present in the seeds deposited under accession number NCIMB 42541 or NCIMB. ____.
E7. The plant cell of any of E1-E6 wherein the plant cell is a seed cell.
E8. Use of a plant comprising the cell of any of E1-E7 to cross with another tomato plant and optionally select progeny from said crossing.
E9. Use of the mutation of the plant cell of E1-E7 to identify tomato plants conferring resistance to to fungi of the division *Ascomycota* or of the family *Sclerotiniaceae,* or to *Botrytis cinerea.*
A1. A cultivated plant of the species *Solanum lycopersicum* comprising a *dmr6* allele encoding the DMR6 protein of SEQ ID No: 1 or a variant of SEQ ID No: 1 comprising at least 77% sequence identity to SEQ ID No: 1, wherein said DMR6 protein or variant comprises one or more mutations of an amino acid selected from amino acids 144 to 187 of SEQ ID No: 1 or the corresponding amino acids in said variant of SEQ ID No: 1.
A2. The cultivated plant of embodiment A1, wherein said one or more mutations is a single mutation.
A3. The cultivated plant of any of A1-A2, wherein the mutation is a substitution.
A4. The cultivated plant of any of A1-A3, wherein said DMR6 protein or variant comprises a mutation at amino acid position 165 of SEQ ID No: 1 or at amino acid position 185 of SEQ ID No: 1, or the corresponding amino acid positions in a variant of SEQ ID No: 1.
A5. The cultivated plant of A4, wherein said mutation at amino acid position 165 of SEQ ID No: 1 is an arginine to lysine substitution (R165K), or wherein said mutation at amino acid position 185 of SEQ ID No: 1 is a leucine to phenylalanine substitution (L185F).
A6. The cultivated plant of A1-A5 wherein said plant comprises a nucleic acid molecule which comprises a sequence selected from the group consisting of SEQ ID NO: 6, SEQ ID NO: 7, a sequence hybridizing under stringent hybridisation conditions to SEQ ID NO: 6, a sequence hybridizing under stringent hybridisation conditions to SEQ ID NO: 7, a sequence identical to SEQ ID No: 6 for all but 20 nucleotides, and a sequence identical to SEQ ID No: 7 for all but 20 nucleotides.
A7. The cultivated plant of any of A1-A6, wherein said *dmr6* allele is endogenous.
A8. The cultivated plant of any of A1-A7, wherein said *dmr6* allele is the allele present in the seeds deposited under accession number NCIMB 42541 or NCIMB____.
A9. The cultivated plant according to A1-A8, wherein the cultivated plant is an F1 hybrid plant.
A10. The cultivated plant of any of A1-A9, wherein said *dmr6* allele is present in homozygous form.
A11. The cultivated plant of any of A1-A10, wherein said dmr6 protein confers resistance to fungi of the division *Ascomycota* or of the family *Sclerotiniaceae*, or to *Botrytis cinerea.*
A12. A seed from which a plant according to A1-A11 can be grown.
A13. A part of the cultivated plant of anyone of A-A11, comprising the *dmr6* allele.
A14. The part of the cultivated plant of A13, wherein said part is selected from the group consisting of a fruit, a part of a fruit, a seed, a seed coat, a leaf, a leaflet, a part of a leaf, a part of a leaflet, a cotyledon, a hypocotyl, a flower, a part of a flower, a root, a part of a root, a stem, a part of a stem, pollen, an ovary, an ovule, an oocyte, an anther, a cutting, a cell, a non-propagating cell, a tissue, an organ, progeny, a protoplast, a rootstock, a scion, and a graft, wherein said part comprises the dmr6 allele of anyone of A1-A11.
A15. A method for producing a cultivated *Solanum lycopersicum* plant, comprising the steps of
   a. crossing the plant of anyone of A1-A11 with a second *Solanum lycopersicum* plant to obtain a progeny seed;
   b. optionally harvesting said progeny seed;
   c. optionally selecting progeny seed comprising the *dmr6* allele of anyone of A1-A11; and
   d. optionally growing the selected progeny of step (c).
A16. A method for reproducing the cultivated *Solanum lycopersicum* plant of anyone of A1 -A11 comprising the step of asexual propagation of the plant of anyone of A1 - A11.
A17. Use of one or more mutations at amino acid positions 144-187 of SEQ ID No: 1 or a variant thereof to identify resistance to a pathogenic fungus selected from the group of *Ascomycota, Sclerotiniaceae* and *Botrytis cinerea* in a *Solanum* plant or part thereof.
A17B. The use of A17 wherein the mutation is at amino acid position acid 165 or 185 of SEQ ID No: 1 or a variant thereof.
A17B2 Use of claim A17B wherein the mutation is a R165K, or wherein said mutation a L185F mutation.
A17C. The use of claim A17 or A17 B wherein the pathogenic fungus is *Botrytis cinerea.*
A17D. The use of claim A17, A17B or A17C wherein the *Solanum* plant is a *Solanum lycopersicum* plant.
A18. A DMR6 protein of SEQ ID No: 1 or a variant thereof, comprising at least 77% sequence identity to SEQ ID No: 1, wherein said DMR6 protein or variant thereof comprises one or more mutations at amino acid position 144 to 187 of SEQ ID No: 1 or the corresponding amino acid position in a variant of SEQ ID No: 1; or the DMR6 protein of SEQ ID No: 2 or a variant thereof comprising at least 77% sequence identity to SEQ ID No: 2 and comprising a Lysine at position 165; or the DMR 6 protein of SEQ ID No: 3 or a variant thereof comprising at least 77% sequence identity to SEQ ID No: 3 and comprising a phenylalanine at position 185.
A19. An isolated nucleic acid molecule encoding the protein of A18; or an isolated nucleic acid molecule comprising any of the sequences selected from the group consisting of SEQ ID No: 6, SEQ ID No: 7. SEQ ID NO: 8 and SEQ ID NO: 9.
A20. A method to identify resistance to a pathogenic fungus selected from the group of *Ascomycota, Sclerotiniaceae* and *Botrytis cinerea* in a *Solanum lycopersicum* plant or part thereof comprising the step of identifying at least one of the nucleic acids of claim 19 in said plant or part thereof.
A20A. A method to identify resistance to a pathogenic fungus selected from the group of *Ascomycota, Sclerotiniaceae* and *Botrytis cinerea* in a *Solanum lycopersicum* plant or part thereof comprising the step of identifying at least one of the proteins of claim 18 in said plant or part thereof.
A21. The method of A20 further comprising the step of selecting the plant or part thereof comprising said nucleic acid, and, optionally, growing fruits from said plant.
A21A. The method of A20A further comprising the step of selecting the plant or part thereof comprising said nucleic acid, and, optionally, growing fruits from said plant.

### Seed Deposits

A representative sample of seed of the *Solanum lycopersicum* dmr6 mutant (dmr6 R165K mutant) according to Example 1, was deposited by Nunhems B.V. and accepted for deposit on 9 February 2016 at the NCIMB Ltd. (Ferguson Building, Craibstone Estate, Bucksburn Aberdeen, Scotland AB21 9YA, UK) according to the Budapest Treaty, under the Expert Solution (EPC 2000, Rule 32(1)). The seed of the dmr6 R165K mutant was given the following deposit numbers: NCIMB 42541 (*Solanum lycopersicum* R150070).

A representative sample of seed of the *Solanum lycopersicum* dmr6 mutant (dmr6 L185F mutant) according to Example 1, was deposited by Nunhems B.V. and accepted for deposit on ______ at the NCIMB Ltd. (Ferguson Building, Craibstone Estate, Bucksburn Aberdeen, Scotland AB21 9YA, UK) according to the Budapest Treaty, under the Expert Solution (EPC 2000, Rule 32(1)). The seed of the dmr6 L185F mutant was given the following deposit numbers: NCIMB ________ (*Solanitm lycopersicum*_____).

The Applicant requests that samples of the biological material and any material derived from said samples be only released to a designated Expert in accordance with Rule 32(1) EPC or related legislation of countries or treaties having similar rules and regulation, until the mention of the grant of the patent, or for 20 years from the date of filing if the application is refused, abandoned, withdrawn or deemed to be withdrawn.

Access to the deposit will be available during the pendency of this application to persons determined by the Director of the U.S. Patent Office to be entitled thereto upon request. Subject to 37 C.F.R. § 1.808(b), all restrictions imposed by the depositor on the availability to the public of the deposited material will be irrevocably removed upon the granting of the patent. The deposit will be maintained for a period of 30 years, or 5 years after the most recent request, or for the enforceable life of the patent whichever is longer, and will be replaced if it ever becomes nonviable during that period. Applicant does not waive any rights granted under this patent on this application or under the Plant Variety Protection Act (7 USC 2321 et seq.).

### Examples

### General methods & materials

PCR amplification products were directly sequenced by a service company (BaseClear, The Netherlands, http://www.baseclear.com/) using the same primers as were used for the amplification. The obtained sequences were aligned using a computer program (CLC Bio Main Work Bench, Denmark, www.clcbio.com) to identify the nucleotide changes.

### Materials

The *Botrytis cinerea* used in these examples was a field isolate from California, USA, maintained in the company for several years. The skilled person is aware of methods for identifying and isolating *Botrytis cinerea.* Any California field isolate of *Botrytis cinerea* can be used in these tests.

Water used for analyses is tap water filtered in an Milli-Q water Integral system, Milli-Q type Reference A+ supplied with a Q-gard T2 Cartridge and a Quantum TEX Cartridge (MQ water). Water resistance is >= 18 MOhm.

Ethyl Methanesulfonate (EMS) (pure) was obtained from Sigma, product number M0880.

Broth for *Botrytis cinerea* comprises 24 g/L potato dextrose broth + 1g/L yeast extract in water.

### EXAMPLE 1

### Mutagenesis

DNA was isolated from a population of individual plants descended from a highly homozygous inbred line used in commercial processing tomato breeding by pooling 10 seeds collected from a fruit.

Grinding ten *S. lycopersicum* seeds produced enough DNA to saturate the magnetic beads, thus highly homogenous and comparable DNA concentrations of all samples were obtained. Comparing with λ DNA references, a concentration of 30 ng/µl for each sample was estimated. Two times diluted DNA was 4 fold flat pooled. 2 µl pooled DNA was used in multiplex PCRs for mutation detection analysis.

Primers were designed to the regions of interest using a computer program (Primer3, available through the world wide web at primer3.sourceforge.net) and HRM (High Resolution Melting; a non-enzymatic screening technique proven to be sensitive and high-throughput methods in human and plant genetics) analysis performed on 4X pooled DNAs of the population using the primers (SEQ ID NO:10 DMR6_EX3_F: 5'-tcgaagaattaatgtaaatagtcgttg-3', SEQ ID NO:11 DMR6_EX3_R: 5'-cgatttgaaggagttgtggtt-3', 382 bp amplicon product). Quality of the primers was determined by a test PCR reaction that should yield a single product.

The Polymerase Chain Reaction (PCR) to amplify gene fragments was done according to the following protocol: 10ng of genomic DNA was mixed with 4µl reaction buffer (5x Reaction Buffer), 2µl 10xLC dye ((LCGreen+ dye, Idaho Technology Inc., UT, USA), 5pmole of forward and reverse primers each, 4nmole dNTPs (Life Technologies, NY, USA) and 1 unit DNA polymerase (Hot Start II DNA Polymerase) in a total volume of 10µl, under reaction conditions 30s 98°C, then 40 cycles of 10s. 98°C, 15s 60°C, 25s of 72°C and finally 60s at 72°C.

HRM curve analysis was performed as follows: During the PCR amplification dye (LCGreen+ dye, Idaho Technology Inc., UT, USA) molecules intercalate between each annealed base pair of the double stranded DNA molecule. When captured in the molecule, the dye emits fluorescence at 510 nm after excitation at 470 nm. A camera in a fluorescence detector (LightScanner, Idaho Technology Inc., UT, USA) records the fluorescence intensity while the DNA sample is progressively heated. At a temperature dependent on the sequence specific stability of the DNA helices, the double stranded PCR product starts to melt, releasing the dye. The release of dye results in decreased fluorescence that is recorded as a melting curve by the fluorescence detector. Pools containing a mutation form hetero duplexes in the post-PCR fragment mix. These are identified as differential melting temperature curves in comparison to homo duplexes.

The presence of the particular mutation in individual plants was confirmed repeating the HRM analysis on DNA from the individual M2 seed lots of the identified corresponding DNA pool. When the presence of the mutation, based on the HRM profile, was confirmed in one of the four individual M2 family DNA samples, the PCR fragments were sequenced to identify the mutation in the gene. Positive pools were de-convoluted by HRM screens of the individuals comprising the pools. Direct sequencing was performed on the candidate mutant individuals to validate the presence of a mutation.

A multi-species alignment (MSA) was performed to determine the highly conserved protein blocks in DMR6. Once the mutation was known the effect of such an mutation was predicted using a computer program CODDLe (for Choosing codons to Optimize Discovery of Deleterious Lesions, available through the world wide web at proweb.org/coddle) that identifies the region(s) of a user-selected gene and of its coding sequence where the anticipated point mutations are most likely to have an effects on the gene's function.

### Mutant identification

Identified mutations were evaluated using standard bioinformatics methods to identify the subset leading to amino acid changes. Mutants containing altered amino acids were evaluated against the MSA to estimate the subset causing a potential functional impairment and the mutant S1DMR6_R165K causing an R to K substitution at amino acid 165 of the tomato DMR6 protein and S1DMR6_L185F causing an L to F substitution at amino acid 185 of the tomato DMR6 protein (SEQ ID NO: 1) were chosen.

Seeds from M2 families comprising mutations with predicted effect on protein activity were sown for phenotypic analysis of the plants. In addition, plants from these seeds comprising the mutation were grown and genotyped to validate the presence of the mutation. The mutation was fixed in a homozygous state by selfing and a seed increase was performed. The effect of the mutation on the corresponding protein and phenotype of the plant was determined.

The protein sequences of the identified mutants are shown as SEQ ID No: 2 (DMR6 R165K) and SEQ ID No: 3 (DMR6 L185F), the genomic DNA sequences as SEQ ID No: 7 (DMR6 R165K) and SEQ ID No: 9 (DMR6 L185F), the cDNA sequences as SEQ ID No: 6 (DMR6 R165K) and SEQ ID No: 8 (DMR6 L185F).

### Disease resistance evaluation

Seeds containing the identified mutation were germinated and plants were grown in pots with soil the greenhouse with 16/8 h light dark regime and 18°C night and 22-25 °C day temperature. Five plants each of DMR6-R165K and the susceptible control (commercial hybrid variety Shady lady) were raised to approximately 8 weeks. The plants were arranged in a randomized compete block design.

Five consecutive petioles at the upper part of the plant were cut off from each plant with a clean sharp implement, to create five inoculation points per plant. Each point was inoculated with a spore population of the field isolate *Botrytis cinerea* kept in-company.

The plants were evaluated 14 days after inoculation, or when sporulation of *Botrytis cinerea* started if that occurred earlier. Start of sporulation before 14 days was considered an indication of severe infection and thus low resistance, and so were lesions that circumfered the stem. Lesion size was measured by determining the length of the lesion surrounding the inoculation along the stem using digital calipers. If a healthy petiole was present, its diameter was measured and subtracted from the total lesion length (Length disease = (lesion length **-** diameter healthy petiole). Typical examples of a lesions on a *Botrytis cinerea* resistant plant and a susceptible plant are shown in Figure 1.

The lesion size as recorded on the aforementioned plants, as an indication of *Botrytis cinerea.* resistance, is shown in the table below

**Table 1 Lesion length (Length disease = (lesion length - diameter healthy petiole)**

| **Cross** | **Unique ID** | **lesion length (mm)** | **Average (mm)** |
|---|---|---|---|
| DMR6-R165K | Mutant | 2.42 | 2.94 |
| | | 1.38 | |
| | | 5.83 | |
| | | 0.00 | |
| | | 6.88 | |
| | | 1.10 | |
| Shady lady | check | 14.57 | 17.13 |
| | | 18.62 | |
| | | 14.40 | |
| | | 13.22 | |
| | | 17.23 | |
| | | 24.73 | |

As can be seen, the plants of the invention (e.g. DMR6 R165) exhibit shorter/lower lesion length/size or even absence of lesions compared to Shady lady, a wild type commercial check.

Similar results were obtained for the DRM6 L185F mutant.

### References

Egashira H, Kuwashima A, Ishiguro H, Fukushima K, Kaya T, Imanishi S (2000) Screening of wild accessions resistant to gray mold (Botrytis cinerea. Pers.) in Lycopersicon. Acta Physiol Plant 22:324-326
Finkers R, van den Berg P, van Berloo R, ten Have A, van Heusden AW, van Kan JA, Lindhout P., 2007 Three QTLs for Botrytis cinerea resistance in tomato Theor Appl Genet. 114(4):585-93.
Victor Flors, Maria de la O. Leyva, Begonya Vicedo, Ivan Finiti, Maria Dolores Real, Pilar Garcia-Agustin, Alan B. Bennett and Carmen Gonza lez-Bosch The Plant Journal (2007) 52, 1027-1040 doi: 10.1111/j.1365-313X.2007.03299.x Absence of the endo-b-1,4-glucanases Cel1 and Cel2 reduces susceptibility to Botrytis cinerea in tomato
George and Heringa, (2002) Protein Eng. 15 (11): 871-879
Henikoff & Henikoff, 1992, PNAS 89, 915-919
Kiyoshi Imada, Shuhei Tanaka, Yukihiro Masuda, Tetsuya Maekawa, and Shin-ichi Ito. Physiological and Molecular Plant Pathology Jan 2015, Volume 89, 1-7 Induction of disease resistance against Botrytis cinerea in tomato (Solanum lycopersicum L.) by using electrostatic atomized water particles
Leroux PI, Fritz R, Debieu D, Albertini C, Lanen C, Bach J, Gredt M, Chapeland F. Pest Manag Sci. 2002;58(9):876-88.Mechanisms of resistance to fungicides in field strains of Botrytis cinerea.
Martínez-Hidalgo P, Garcia JM, Pozo MJ. Front Microbiol. 2015 6:922. doi: 10.3389/fmicb.2015.00922. eCollection 2015. Induced systemic resistance against Botrytis cinerea by Micromonospora strains isolated from root nodules.
Nicot, P. C, Moretti A.,Romiti, C, Bardin, M., Caranta C, Ferriere H. INRA Report of the Tomato Genetics Cooperative Number 52 - September 2002. Differences in susceptibility of pruning wounds and leaves to infection by Botrytis cinerea among wild tomato accessions.
O'Neill, T.M.; Shtienberg, D.; Elad, Y. Effect of some host and microclimate factors on infection of tomato stems by Botrytis cinerea. Plant Disease, Saint Paul, v. 81, n. 1, p.36-40, 1997
Van Damme et al., 2005 Mol Plant Microbe Interact. 2005 Jun;18(6):583-92. Identification of arabidopsis loci required for susceptibility to the downy mildew pathogen Hyaloperonospora parasitica.
Van Damme et al., 2008, The Plant Journal 54, 785-793
Urbasch, I. 1986 Journal of Phytopathology 116, p 344-351, Resistenz verschiedener Kultur- und Wildtomatenpflanzen (Lycopersicon spp.) gegenüber Botrytis cinerea Pers. DOI: 10.1111/j.1439-0434.1986.tb00930.x
Sambrook et al. (1989) Molecular Cloning: A Laboratory Manual, Second Edition, Cold Spring Harbor Laboratory Press
Sambrook and Russell (2001) Molecular Cloning: A Laboratory Manual, Third Edition, Cold Spring Harbor Laboratory Press, NY
Zeilmaker (2012) Negative regulation of plant defence by the oxygenase DMR6 acts upstream of salicylic acid accumulation. Chapter 4 of Utrecht University thesis Functional and applied aspects of the DOWNY MILDEW RESISTANT 1 and 6 genes in Arabidopsis.
Zeilmaker et al., Plant J. 2015, 81(2): p210-22
Zeilmaker (thesis) Functional and applied aspects of the DOWNY MILDEW RESISTANT 1 and 6 genes in Arabidopsis 2012 Chapter 4
http://www.micronic.com/
http://www.vaskon.com/
http://www.perkinelmer.com/
http://tilling.ucdavis.edu/index.php/Tomato_Tilling
Till et al. 2007, BMC Plant Biol 7: 19
Till et al. 2006, Methods Mol Biol 323: 127-35
Till et al. 2004, BMC Plant Biol 4: 12
http://www.thermoscientific.com

## Claims

1. A cultivated plant of the species *Solanum lycopersicum* comprising a *dmr6* allele encoding the DMR6 protein of SEQ ID No: 1 or a variant of SEQ ID No: 1 comprising at least 77% sequence identity to SEQ ID No: 1, wherein said DMR6 protein or variant comprises one or more mutations of an amino acid selected from amino acids 144 to 187 of SEQ ID No: 1 or the corresponding amino acids in said variant of SEQ ID No: 1.

2. The cultivated plant of claim 1, wherein said one or more mutations is a single mutation, optionally wherein the mutation is a substitution.

3. The cultivated plant of any of the preceding claims, wherein said DMR6 protein or variant comprises a mutation at amino acid position 165 of SEQ ID No: 1 or at amino acid position 185 of SEQ ID No: 1, or the corresponding amino acid positions in a variant of SEQ ID No: 1.

4. The cultivated plant of claim 3, wherein said mutation at amino acid position 165 of SEQ ID No: 1 is an arginine to lysine substitution (R165K), or wherein said mutation at amino acid position 185 of SEQ ID No: 1 is a leucine to phenylalanine substitution (L185F).

5. The cultivated plant of any of the preceding claims wherein said plant comprises a nucleic acid molecule which comprises a sequence selected from the group consisting of SEQ ID NO: 6, SEQ ID NO: 7, a sequence hybridizing under stringent hybridization conditions to SEQ ID NO: 6, a sequence hybridizing under stringent hybridization conditions to SEQ ID NO: 7, a sequence identical to SEQ ID No: 6 for all but 20 nucleotides, and a sequence identical to SEQ ID No: 7 for all but 20 nucleotides.

6. The cultivated plant of any of the preceding claims, wherein said *dmr6* allele is endogenous.

7. The cultivated plant of any of the preceding claims, wherein said *dmr6* allele is the allele present in the seeds deposited under accession number NCIMB 42541 or NCIMB____.

8. The cultivated plant according to any one of the preceding claims, wherein the cultivated plant is an F1 hybrid plant.

9. The cultivated plant of any of the preceding claims, wherein said *dmr6* allele is present in homozygous form.

10. The cultivated plant of any of the preceding claims, wherein said dmr6 protein confers resistance to fungi of the division *Ascomycota* or of the family *Sclerotiniaceae*, or to *Botrytis cinerea.*

11. A seed from which a plant according to any one of the preceding claims can be grown.

12. A part of the cultivated plant of anyone of claim 1-11, comprising the *dmr6* allele, optionally wherein said part is selected from the group consisting of a fruit, a part of a fruit, a seed, a seed coat, a leaf, a leaflet, a part of a leaf, a part of a leaflet, a cotyledon, a hypocotyl, a flower, a part of a flower, a root, a part of a root, a stem, a part of a stem, pollen, an ovary, an ovule, an oocyte, an anther, a cutting, a cell, a non-propagating cell, a tissue, an organ, progeny, a protoplast, a rootstock, a scion, and a graft, wherein said part comprises the dmr6 allele of anyone of claim 1-11.

13. A method for reproducing the cultivated *Solanum lycopersicum* plant of anyone of claim 1 -10 comprising the step of asexual propagation of the plant of anyone of claim 1-10.

14. A DMR6 protein of SEQ ID No: 1 or a variant thereof, comprising at least 77% sequence identity to SEQ ID No: 1, wherein said DMR6 protein or variant thereof comprises one or more mutations at amino acid position 144 to 187 of SEQ ID No: 1 or the corresponding amino acid position in a variant of SEQ ID No: 1; or the DMR6 protein of SEQ ID No: 2 or a variant thereof comprising at least 77% sequence identity to SEQ ID No: 2 and comprising a Lysine at position 165; or the DMR 6 protein of SEQ ID No: 3 or a variant thereof comprising at least 77% sequence identity to SEQ ID No: 3 and comprising a phenylalanine at position 185; or an isolated nucleic acid molecule encoding any of these proteins; or an isolated nucleic acid molecule comprising any of the sequences selected from the group consisting of SEQ ID No: 6, SEQ ID No: 7. SEQ ID NO: 8 and SEQ ID NO: 9.

15. A method to identify resistance to a pathogenic fungus selected from the group of *Ascomycota, Sclerotiniaceae* and *Botrytis cinerea.* in a *Solanum lycopersicum* plant or part thereof comprising the step of identifying at least one of the nucleic acids of claim 16 in said plant or part thereof.
